# EUROPEAN PATENT SPECIFICATION

(11) **EP 0 405 696 B1**
(45) Date of publication and mention of the grant of the patent: **29.03.1995**
(21) Application number: 90201740.9
(22) Date of filing: 29.06.1990
(51) Int. Cl.: C12N 15/87, C12M 1/00, C12M 3/00, A61K 47/48

(54) **A method and apparatus for inserting material into biological cells**
Methode und Apparat, um Material in biologische Zellen einzufügen
Méthode et appareil pour l'insertion de matériau dans une cellule vivante

(30) Priority: 30.06.1989 US 374406; 09.02.1990 US 477688
(43) Date of publication of application: 02.01.1991
(73) Proprietor: DOWELANCO, Indianapolis, Indiana 46268-3030 (US)
(72) Inventor: Miller, Theodore E., Jr., Midland, Michigan 48640 (US); Schuchardt, Bradley C., Midland, Michigan 48640 (US); Gould, Alan R., Midland, Michigan 48640 (US); Skokut Thomas A., Midland, Michigan 48640 (US)
(74) Representative: Smulders, Theodorus A.H.J., Ir.

(56) References cited:
- EP-A- 0 137 504
- PARTICULATE SCIENCE AND TECHNOLOGY vol. 5, 1987, pages 27 - 37; SNFORD,J.C. et al :Delivery of substances into cells and tissues using a particle bombardment process"

## Description

The present invention is directed to an apparatus and method for the acceleration of propellable matter. More specifically, the present invention is directed to an apparatus and method for contacting a predetermined volume of gas under pressure with a predetermined volume of a suspension of propellable matter, and, thereafter, the suspension is accelerated at selected target matter.

Biologists commonly wish to introduce a wide range of biological material into living cells. There exists much current research directed to the genetic transformation of living cells. Conventional technologies for introducing biological material into living cells include electroporation, direct DNA uptake mechanisms, fusion mechanisms, microinjection mechanisms, and the use of infectious agents. However, each of these techniques suffer from certain practical disadvantages.

Electroporation is a method for introducing a variety of molecules into cells by subjecting them to brief high-voltage electric pulses. For a general discussion of electroporation, see Shigekawa, *et al*. (1988), Biotechniques, **6**:742; Miller, *et al*. (1988), Proc. Natl. Acad. Sci. USA, **85**:856-860; and Powell, et al. (1988), Appl. Environ. Microbiol., **54**:655-660.

General limitations of electroporation include (i) the reduction in overall cell viability caused by high applied voltages, and (ii) the inability to specifically target particular cells, especially in a complex multicellular organ.

Moreover, while electroporation methods have greatly increased the efficiency of uptake of chimeric gene constructions, such methods when used with plants are limited in plants to *in vitro* suspension systems. Moreover, although successfully used in the transformation of monocots as well as dicots (Fromm, *et al*. (1985), Proc. Natl. Acad. Sci. USA, **82**:5824-5828), electroporation methods generally require the relatively laborious and time-consuming step of removing the plant cell walls.

Uptake mechanisms generally involve suspensions of single cells, and specifically when applied to plant cells, require enzymatic removal of cell wall materials. Consequently, the uptake mechanisms are time consuming and have relatively low throughput.

One technique for uptake is the enhancement of membrane permeability, such as by using calcium (Ca) ( Mandel, *et al*. (1972), J. Mol. Biol., **53**:159-162); and temperature shock (Dityatkin, *et al*. (1972), Biochimica et Biophysica Acta, **281**:319-323).

A second technique for uptake is the use of surface-binding agents such as polyethylene glycol (PEG). For a general discussion of the use of surface-binding agents, see Chang, *et al*.(1972), Mol. Gen. Genet., **168**:111-115; Krens, et al. (1982), Nature, **296**:72); or such as calcium phosphate (Graham, *et al*. (1973), Virology, **52**:456; Wigler, *et al*. (1979), Cell, **16**:777.

A third technique for uptake is the phagocytosis of particles into a cell. Suitable particles include liposomes (Uchimiya, *et al*. (1982), In: Proc. 5th Intl. Cong. Plant Tissue and Cell Culture, Fujiwara (ed.), pp. 507-508); organelles (Potrykus (1973), Z. Pflanzenphysiol., **70**:364-366); or bacteria (Cocking (1972), Ann. Rev. Plant Physiol., **23**:29-50).

Fusion mechanisms incorporate new genetic material into a cell by fusing a cell membrane with the membrane of another cell, an organelle, or a liposome. As with uptake mechanisms, plant cell fusion technologies rely upon the use of *in vitro* suspension systems, where cells are enzymatically stripped of any cell wall material.

Fusion can be induced with electric currents, PEG, and Sendai virus particles. For a general discussion of cell fusion, see Uchidaz, *et al*. (1980), In: Introduction of Macromolecules Into Viable Mammalian Cells, Baserga, *et al*. (eds.), **1**:169-185; and Harris (1970), Cell Fusion: The Dunham Lectures.

While fusion technologies can have relatively good efficiencies in terms of numbers of cells affected, the problems of cell selection can be complex. For example, in the case of cell to cell fusion the resulting cells often have elevated ploidy, which can limit their usefulness.

Microinjection is a direct method for the transfer of chromosomes by microinjection. Microinjection techniques employ extremely fine, drawn-out capillary tubes which can be used as syringe needles for the direct injection of biological substances into certain types of individual cells. When small cells need to be injected, very sharp capillaries, whose tips are very easily broken or clogged, are required. Moreover, very high pressures are required to cause bulk flow through capillary apertures smaller than one micron and the regulation of such bulk flow can be difficult. The entire process is rather empirical, requiring different modifications for different cell types.

For a general discussion of microinjection techniques, see Diacumakos (1973), In: Methods in Cell Biology, Prescott (ed.), pp. 287-311; Graessman and Graessman, Methods in Enzymology, **101**:482-492; Crossway, *et al*. (1986), Mol. Gen. Genet., **202**:179-185; Crossway, *et al*. (1986), Biotechniques, **4**:320-334; Reich, *et al*. (1986), Can. J. Bot.; and Reich, *et al*. (1986), Bio/Technology, **4**:1001-1004.

Microinjection techniques suffer from limitations in cell recovery. Direct microinjection of plant cells is further complicated by the presence of a rigid cell wall. While protoplasts lacking the cell wall can be formed, the microinjection of plant cell protoplasts is made difficult by their extreme fragility.

Thus, a disadvantage of microinjection is that it requires single cell manipulations and is, therefore, inappropriate for treating masses of cells. The process is generally very tedious and difficult. Consequently, it tends to have very low efficiency and low throughput.

In addition to the systems mentioned above, there exist several infectious agents which can deliver nucleic acids into cells. The plant pathogen *Agrobacterium tumefaciens* has the innate ability to transfer a portion of DNA from a Ti (Tumor-inducing) plasmid harbored therein into an infected plant cell. By inserting foreign genes into plasmids in *Agrobacterium* which carry certain sequences from the Ti plasmid, the bacterial transformational trait can be used to transport the foreign genes into the genome of the infected plant cells.

Of primary importance are the *Agrobacterium* vectors for dicot plant cells (Fraley, *et al*. (1986), CRC Crit. Rev. Plant Sci., **4**:1-46); and the retroviral vectors for animal cells (Anderson (1984), Science, **226**:401-409).

Retroviruses (RNA viruses) can be used to deliver genes into animal cells. When the virus enters the cell its RNA acts as a template for reverse transcription of complementary DNA which will integrate into the genome of the host cell. This DNA can be isolated and inserted into a plasmid. This plasmid, with additional genes added, can be used to transform cells with the aid of helper retroviruses.

However, these systems are frequently difficult to control. The problem with using infectious agents such as DNA delivery systems is several-fold. First, infectious agents have limited host ranges. The mediation can only be done on an individual cellular level, typically with somatic tissues, which then must be regenerated artificially into a whole plant. This limits the applicability of *Agrobacterium*-mediated genetic transformation to those crop species which can readily be regenerated from types of tissues which are susceptible to *Agrobacterium* infection; the natural host range of *Agrobacterium* includes only dicotyledonous plants and a limited number of monocot species of the *Liliaceae* family. Likewise, retroviruses, and the expression of the DNA that they deliver, tend to be host and tissue specific.

Second, infectious agents add an additional level of complexity to the delivery process by introducing a second living system with all its concomitant complications. For example, *Agrobacterium*-mediated transformations may generate somoclonal variants, which spontaneously arise in plant tissues in tissue culture and which may complicate identification of transformants. In addition, infectious agents such as retroviruses are potentially dangerous - they may harm the organism being modified, or they may lead, through recombination, to the evolution of new pathogens.

Relatively recently, Sanford *et al*. developed a method whereby substances can be delivered into cells of intact tissues. (Klein, *et al*. (1987), Nature, 337; and Sanford, *et al*. (1988), Particular Sci. and Technol., **5**:27-37).

These references teach that small, high-density, tungsten particles (microprojectiles) may be accelerated to high velocity by a particle gun apparatus. Sanford *et al*. teach that they have accelerated microprojectiles to sufficient velocities to allow plant cell penetration via the following embodiments: (1) a macroprojectile (plastic bullet) and stopping plate, (2) a transferred mechanical pulse, (3) a gas (e.g., air) discharge, and (4) a centripetal acceleration system.

While the particle gun apparatus represents a proposed advance in the art, its various embodiments of the particle bombardment process have certain deficiencies. For example, in each of the various embodiments, the tungsten load suspension amount is not easily reproducible; and it is a rather laborious procedure to inoculate cultures repeatedly. Additionally, in the macroprojectile embodiment (1) and the gas discharge embodiment (3), the velocity must vary with inevitable differences in the firing characteristics; and the suspension velocity is not directly measured. Finally, the macroprojectile embodiment (3) has the following additional deficiencies: the plastic bullet's inertia impairs velocity - making a powerful explosion necessary; the velocities are not easily changed or controlled; and the combustion gases from the gunpowder could be problematical.

Therefore, the development of a technique that can efficiently deliver noncellular biological material directly into living cells and tissues would be beneficial.In one aspect, the present invention provides an apparatus comprising (a) a source of gas under pressure having an outlet; (b) a propellable matter reservoir having an inlet and an outlet; and (c) a multipurpose valve providing selective communication between the outlet of the source of gas under pressure and the inlet of the propellable matter reservoir. In a second aspect, the present invention provides a method for introducing biological material into target matter, said method comprising the following steps: (a) providing a predetermined volume of gas in a gas reservoir at predetermined pressures; (b) providing, in a propellable matter reservoir, a predetermined quantity of a propellable matter, which comprises a suspension of a biological material in a carrier medium, said reservoir having a reservoir inlet in selective communication with the gas outlet, and a reservoir outlet in selective communication with the overflow inlet and the delivery inlet; (c) contacting said propellable matter with the predetermined volume of a gas; and (d) accelerating the propellable matter through a delivery means at the target matter.

Further features and advantages of the present invention will become more apparent from the following specification taken in connection with the drawings wherein:
Figure 1 is a schematic representation of an apparatus in accordance with one embodiment of the invention.
Figure 2 is a schematic representation of a valve for use with the apparatus of Figure 1 and in accordance with the invention.
Figure 3 is a schematic representation of an embodiment of a source of gas under pressure for use with Figure 1 and the apparatus of the present invention.
Figures 4A & 4B are schematic representations of various embodiments of a source of propellable matter for use with the apparatus of Figure 1 and in the present invention.
Figures 5A and 5B are schematic representations of a propelling and sighting means for use with an apparatus such as indicated in Figure 1 and in accordance with the invention.
Figure 6 is a schematic representation of an exemplary embodiment of a velocity detection means for use with the present invention.
Figure 7 is a schematic representation of synchronizing means for use with an apparatus such as indicated in Figure 1 and in accordance with the invention.
Figures 8A & 8B are schematic representations of an electric circuit as used with the velocity detection means depicted in Figure 6 hereof, as hereinafter described in the "Examples" section.
Figure 9 is a graphical representation of the raw data used to measure time of flight of the propellable matter by the electronic circuit hereof, as hereinafter set forth in the "Examples" section.

The present invention is generally directed to the method and apparatus of accelerating propellable matter. It should be understood that the invention is not limited to a method or apparatus for the propulsion of a specific material, but rather the present invention may be used for the propulsion of many types of materials. The invention is, however, principally adapted to a method and apparatus for the acceleration of a suspension of biological material.

The biological material may be cellular or noncellular. The biological material may be relatively small, preferably less than 1 micron, more preferably less than 0.1 micron. Generally, the amount of biological material employed will be up to 5 micrograms (»g), with specific amounts possibly varying based upon the type of cellular material used.

By "noncellular biological material" is meant to include viruses (tobacco mosaic virus (TMV), cauliflower mosaic virus (CAMV), maize streak virus (MSV), etc.); organelles (e.g., mitochondria, nucleus, chloroplast or plastid); genetic material, (e.g., either ribonucleic acid (RNA) or deoxyribonucleic acid (DNA), in the form of plasmids or single or double strands); proteins (antibodies or enzymes), or stains.

When DNA is the noncellular biological material, the DNA may be constructed in a vector appropriate for expression of the exogenous gene in the cells. Suitable transformation vectors include expression vectors.

Vectors suitable for expression generally must include, besides the coding sequence of the desired exogenous gene, appropriate flanking regulatory sequences. Such flanking sequences include a suitable promoter capable of promoting transcription and expression *in vivo* in cells and a translation terminator capable of signaling the end of transcription or the appropriate processing of the RNA in such a fashion that will allow suitable translation of messenger RNA for protein synthesis.

When DNA is inserted into living cells, it is preferable to screen the progeny at some stage to select for transformants because not all of the cells will have carrier particles inserted into them and not all cells or progeny will uptake the DNA into their genome. The presence of the desired DNA in the cells can then be established in a wide variety of ways, depending on the nature of the DNA.

The transformation vector may contain a selectable marker to allow for selection of transformed cells. The selectable marker may condition a trait which may be assayed biochemically or a phenotypic trait which may be observed.

Alternatives to the use of such a selectable marker include suitable morphological or biochemical tests to screen for the transformed progeny. A morphological screening test may be for a dominant phenotypic trait in the progeny. A suitable biochemical screening test is a so-called "Southern" blot with a probe hybridizing to the transforming DNA - itself in the genome of the microorganism, plant or animal cells.

The presence of a gene which produces an exogenous product may also be detected by isolation and lysis of the cell and an analysis of the cytoplasm for the exogenous product, or of the nucleus for the exogenous gene. The exogenous product may be detected by electrophoresis, chromatography, immunoassay, Southern blotting or the like.

By "cellular biological material" is meant individual or multicellular masses of cellular microorganisms, plant cells or animal cells.

As used herein, the term "cellular microorganisms" includes bacteria and protozoans.

As used herein, the term "plant cells" includes cells that are intact in a plant or parts of a plant, e.g., flowers, kernels, ears, cobs, leaves, husks, stalks; plant cell or tissue cultures, which are with or without cell walls or other natural protective coating, e.g., protoplasts; plant calli; plant tissue clumps; subcellular structures; pollen grains; plant meristems; eggs; zygotes; seeds capable of being germinated into a plant.

The plant cells may be derived from any plant species. The term "plant species" is meant to include monocotyledons (e.g., the grasses, and the cereal crops such as maize, rye, barley, wheat, sorghum, oats, millet and rice); and the dicotyledons (e.g., broad-leafed plants such as tobacco, potato and alfalfa).

As used herein, the term "animal cells" includes tissue from mammals, fish, birds, reptiles, and amphibians.

The biological material may be adsorbed on the surface of a carrier particle by a variety of techniques. For example, the biological material may be prepared by simply being dried onto suitable carrier particles, as described below.

The carrier particles should have a size, shape, and density sufficient to penetrate the cell membrane and/or cell wall, without causing gross physical damage to the cell. Carrier particles that are too small or not dense enough may fail to penetrate certain cells, while carrier particles that are too large or too dense will be lethal to others. Factors other than cell size, such as the presence of a cell wall or abundance of intracellular nuclei, may also affect the efficiency of transformation by projectiles of a selected size or density.

Generally, the carrier particles have a diameter of between 0.1 micron and 100 microns; preferably between 0.5 micron and 5 microns. Generally, the carrier particles have a density of between 1 gram/centimeter³ (gm/cm³) and 25 gm/cm³; preferably between 10 gm/cm³ and 25 gm/cm³.

The carrier particles may be made of a biologically inert dense material. Exemplary materials include certain metals, e.g., tungsten, gold, platinum, palladium, silver and nickel, latex, glass, ceramic, surgical alloys, and ferrite crystals.

In another embodiment, the carrier particles may be biological material encapsulated by inert materials. An exemplary encapsulating agent is polylysine (molecular weight 200,000). The encapsulating agent is applied to the particles by rinsing the particles in a solution of encapsulating agent and then air drying or heat drying the particles thus coated. Once the carrier particles are coated with an encapsulating agent and have been properly dried, the biological material can then be loaded onto the particles. Alternatively, encapsulation of the biological material could be accomplished in conjunction with precipitation of the material onto the particles.

Additionally, the cellular biological material, either procaryotic or eucaryotic, may be frozen, suspended in the carrier medium, and be used as projectiles for acceleration directly at the target matter.

The invention, however, is not limited to the use of carrier particles with the propelled, biological material. The propelled, biological materials might be frozen, suspended in the carrier medium, and used as projectiles for acceleration directly at the target matter.

Any medium which is not harmful to the propelled, biological material is suitable as a carrier.

The biological matter may be cultured in any medium capable of maintaining the matter and/or sustaining cell metabolism and growth. Exemplary cultures are taught in Murashige and Skoog (1962), Physiol. Plantarum, **15**:473-496; and Schenk and Hildebrandt (1972), Canadian Journal of Botany, **50**: 199-204.

The carrier medium should be selected to have a flow property effective to convey the carrier particles within it. The flow property of the carrier medium is dependent upon the density, surface tension, effective volume, and viscosity of the propellable matter. Preferably, the carrier medium will be capable of suspending the cellular or noncellular biological material, and optionally the carrier particles.

Generally, the carrier medium should have a density which is effective to maintain the particles within it as it moves. Preferably, the density of the carrier medium should be between 0.5 grams per cubic centimeter (g/cc) and 2.0 g/cc.

Generally, the carrier medium should have a surface tension which is effective to maintain its cohesive propellable volume. Preferably, the surface tension of the carrier medium should be between 20 and 80 dynes/cm.

Generally, the carrier medium should have a volume which is effective to suspend a desired number of carrier particles. Preferably, the volume of the carrier medium should be between 0.5 microliter (»l) and 1000 »l, more preferably between 5 »l and 100 »l.

Generally, the carrier medium should have a viscosity which is effective to maintain a cohesive propellable volume and suspend the particles. Preferably, the viscosity of the carrier medium should be between 0.1 centipoise (cp) and 10 cp, most preferably between 0.5 cp and 2 cp.

Exemplary carrier mediums include liquids, such as water, ethanol; buffer solutions, including phosphate, citrate and acetate buffers; salt solutions, including chlorides of potassium, sodium and calcium; glycols, glycerines and fluorinated hydrocarbons; and liquid nitrogen when the cellular or noncellular biological material is frozen.

Suitable target matter may be cellular biological material; or noncellular biological material, each of which is described above. The biological material may be cultured/supported in any medium capable of sustaining the biological material, as described above, supporting it during impact of the microparticles.

A layer of oil may be applied over the cells to control the hydraulic pressure of the medium and to improve cell viability by helping to seal lesions after penetration. For example, the living cells may be bathed in isotonic solution in order to maintain, at the moment the membrane is punctured, the balance of the osmotic pressure of the intercellular matter and the extracellular solution in the zone of the cell membrane puncture. Calcium ions may be present as a membrane stabilizing agent in all these solutions so that the damaged portion of the membrane may quickly be restored. Exemplary isotonic solutions include solutions commonly used for microinjection techniques for plant cells; Ringer solution for cells of cold-blooded animals, protozoa and microorganisms; and Ringer-Locke, Ringer-Tirode and other solutions for cells of animal cells.

Bombardment of the target matter may cause a portion of the sample to be lost due to dispersal upon impact of the propellable matter and target matter. Exemplary support means to hold the target matter in place include filter paper, agar medium, metal screens, and metal screen cages. For example, subsequent incubation and staining of the cellular biological material may be performed directly on the filter paper. In another embodiment, both target matter and propellable matter may be put in solution and bombarded with uncoated carrier particles, which are suspended in the carrier medium. Such carrier particles may pull, in their wake, a given volume of the external solution containing noncellular biological material into the cellular biological material.

A preferred embodiment of the invention will now be described with the reference to the drawings. The apparatus of the present invention may be of any suitable design capable of permitting a predetermined selected volume of gas having a selected gas pressure to contact and accelerate a predetermined amount of a propellable matter towards a selected target matter.

Various embodiments of an apparatus within the scope of the present invention, and set forth in the Figures, are set forth below.

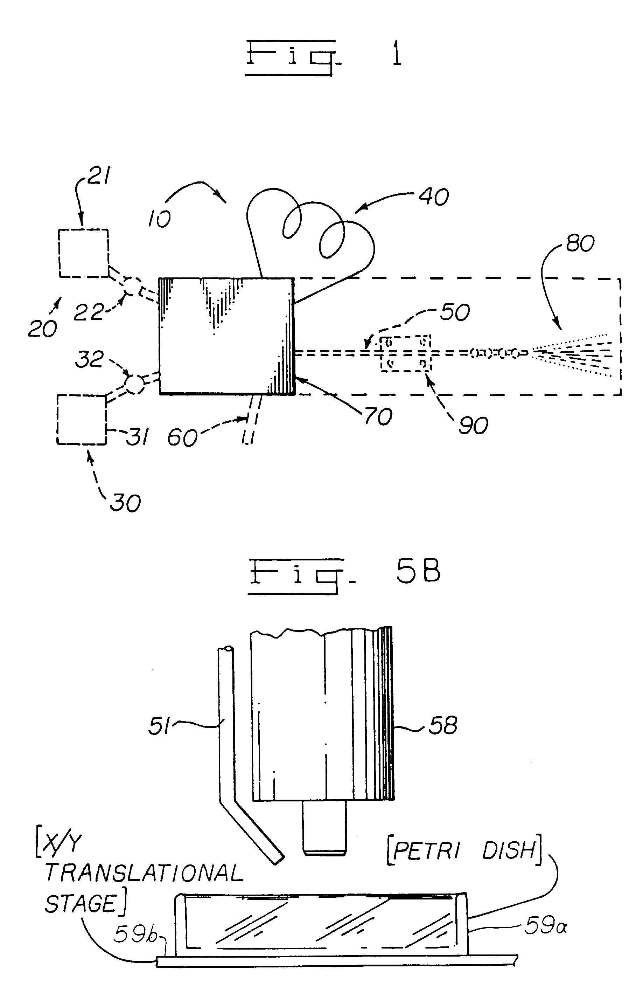

In Figure 1 there is schematically depicted an apparatus, generally designated by the reference numeral 10, which comprises a multipurpose valve 70 having inlets and outlets; a source of gas under pressure 30 having an outlet; a optional source of propellable matter 20 having an outlet; a propellable matter reservoir 40 having an inlet and an outlet, an optional delivery means 50 having an inlet and an outlet; and an optional recovery means 60 having an inlet and an outlet.

Multipurpose valve 70 is selected to have a design capable of being adjusted to provide selective communication between either the source of gas under pressure 30 or, optionally, the source of propellable matter 20, and the propellable matter reservoir 40. Multipurpose valve 70 is also selected to have a design capable of being adjusted to provide selective communication between the propellable matter reservoir 40, and either the delivery means 50 or, optionally, the recovery means 60.

As seen in Figure 2, one embodiment of the multipurpose valve 70 may comprise a first subvalve 70a and a second subvalve 70b. The first subvalve 70a provides selective communication between either the outlet of the source of gas under pressure 30 or the outlet of the source of propellable matter 20, and the inlet of the propellable matter reservoir 40. The second subvalve 70b provides selective communication between the outlet of the propellable matter reservoir 40, and either the inlet of the delivery means 50 or the inlet of the recovery means 60.

Exemplary first subvalve 70a and second subvalve 70b may each be one three-port valve or two two-port valves, provided that the two-port valves optionally are in operative combination via suitable means. Exemplary means for maintaining the two-port valves in operative combination include a common actuator or different actuators (not shown).

The first and second subvalves may in turn be in operative combination via suitable means. Exemplary means for maintaining the two-port valves in operative combination include a common actuator (as shown by 70c) or different actuators (not shown).

The actuators of the first and second subvalves are selected to provide switching of pressurized supplies to the valve. Generally, any actuator capable of producing linear or rotary motion may be used. Exemplary actuators include double-acting pneumatic units operated with rack-and-pinion gearing. When different actuators are used, the actuators may be synchronized by timing means (not shown). Exemplary timing means include multipole switches combined with pilot solenoids or common actuating shafts.

Preferably, the actuators are in operative combination with a foot pedal (not shown), thereby simplifying the operation and increasing the relative throughput of the apparatus.

Generally, the propellable matter reservoir 40 may be selected from any number of containers, such as a tube. The propellable matter reservoir 40 may preferably have an inner surface selected to define an inner space with an aspect ratio sufficient to assure uniform ejection of the propellable matter and to assure minimum hang up, i.e., that which adheres or wets to the inner walls of the propellable matter reservoir 40. Exemplary propellable matter reservoir 40 will be capable of holding between 0.005 milliliter (ml) and 100 ml of propellable matter.

Generally, the propellable matter reservoir 40 will be fabricated of a material physically strong enough to be charged with propellable matter from the source of propellable matter 20 and/or gas from the source of gas under pressure 30, without becoming physically deformed. Preferably, the propellable matter reservoir 40 will be fabricated of a material which is capable of withstanding at least 1000 pounds per square inch (psi) pressure at physiological temperatures. An exemplary material for use in fabricating the propellable matter reservoir is stainless steel.

As seen in Figure 2, the temperature within the propellable matter reservoir 40 may be controlled by temperature control means 42. Exemplary temperature control means include thermocouples implanted in a block heater surrounding the propellable matter reservoir 40.

Although the schematic representation in Figures 1 and 2 show the use of only one source of propellable matter, the principles set forth in the operation of the apparatus 10 may be readily applied to the propulsion of two or more types of propellable matter from a plurality of sources of propellable matter.

Generally, the volume of propellable matter 20 in the propellable matter reservoir 40 may be controlled by any means that provides measured, reproducible samples of propellable matter 20. When tubing or the like is used, one tube capable of holding a first volume may be replaced with another tube capable of holding a second volume. However, the apparatus is not intended to be so limited. For example, a level sensor (not shown) may be positioned at any location effective to provide accurate, reproducible measurements of the volume of propellable matter in the propellable matter reservoir 40.

As seen in Figure 1, the source of gas under pressure 30 may comprise a gas supply means 31, and, optionally, a gas regulating means 32. The source of gas under pressure is selected to provide predetermined volumes of gas into the propellable matter reservoir 40. The gas pressure is dependent upon the type of gas employed and the volume of the gas. Generally, the gas pressure will be between 15 atmospheres (atm) and 150 atm.

The source of gas under pressure 30 may be regulated by any means which is capable of providing gas at a desired pressure into the propellable matter reservoir 40. The gas regulating means may be manually or automatically operated.

Preferably, the source of gas under pressure 30 will be capable of continually providing volumes of gas to the propellable matter reservoir 40, to permit relatively quick, repeated actuations of the apparatus 10.

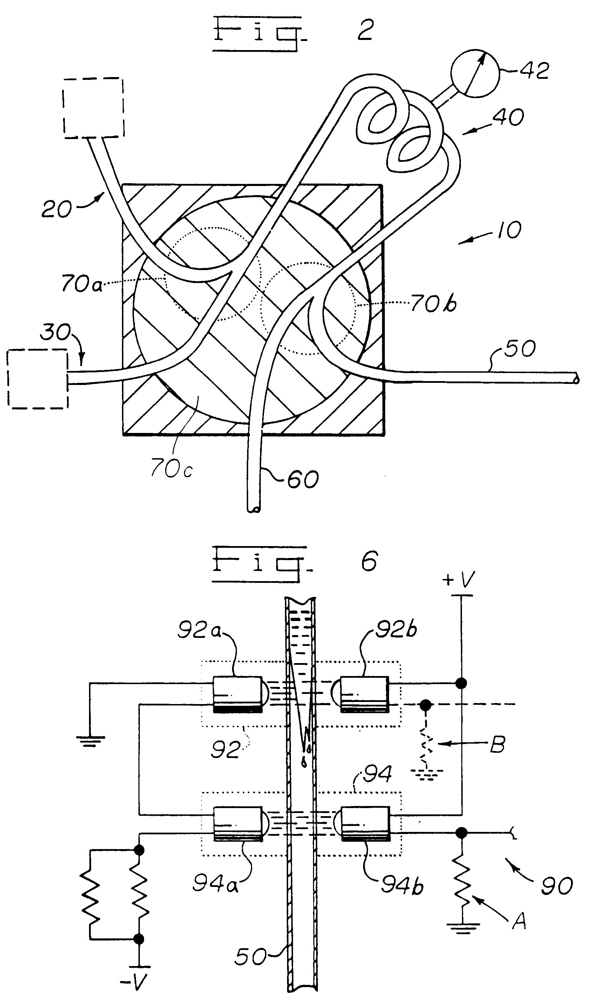

An exemplary source of gas under pressure 30 is further set forth in Figure 3. As shown in Figure 3, the source of gas under pressure 30 comprises (1) a gas supply means 31 in the form of a gas supply tank 31a having an outlet, (2) a gas supply line 33 having an inlet and an outlet; and (3) a gas regulating means 32 in the form of a gas valve 32a, and a gas reservoir 32b having an inlet and an outlet.

The outlet of the gas supply tank 31a is in pneumatic communication with the inlet of the gas supply line 33. The outlet of the gas supply line 33 is in turn in selective pneumatic communication, via gas valve 32a, with the inlet of the gas reservoir 32b.

Exemplary gas supply means 31 include gas cylinders and tanks.

The gas reservoir 32b may be selected from any number of containers, such as tubes. Exemplary gas reservoirs will be capable of holding between 1 ml and 100 ml of gas.

Generally, the gas reservoir 32b may be fabricated of a material physically strong enough to be charged with gas from the source of gas under pressure 30 without becoming physically deformed. Preferably, the gas reservoir 32b will be capable of withstanding up to 150 atm of gas pressure. Exemplary materials for use in fabricating the gas supply line include stainless steel.

The gas should be selected to have a molecular weight sufficiently low to allow rapid enough expansion to produce the requisite particle velocities. Generally, the molecular weight of the gas should be between 2 and 40 atomic mass unit (amu). Exemplary gases include helium, hydrogen and air.

As stated above with reference to Figures 1 and 2, the apparatus preferably comprises a source of propellable matter 20 in selective fluid communication with the propellable matter reservoir 40. When the source of propellable matter 20 is not present, the propellable matter reservoir 40 may be manually filled and affixed in fluid communication with the apparatus.

As seen in Figure 1, and as particularly shown in Figures 4A and 4B, the source of propellable matter 20 is selected to be capable of providing predetermined volumes of propellable matter into the propellable matter reservoir 40.

Preferably, the source of propellable matter 20 will be capable of continually providing selected volumes of propellable matter to the propellable matter reservoir 40, to permit relatively quick, repeated actuations of the apparatus 10. The source of propellable matter 20 comprises a propellable matter supply means 21. Exemplary propellable matter supply means 21 include syringes, tanks, tubing or other conduits.

Generally, the propellable matter supply means 21 will be fabricated of a material which is capable of being sterilized. Exemplary materials include stainless steel and polytetrafluorethylene.

Depending upon the inherent regulating capacity of the propellable matter supply means, the apparatus may comprise means capable of regulating the flow of propellable matter, i.e., propellable matter regulating means 22. The propellable matter regulating means may be in operative combination with the propellable matter supply means 21 to permit a selected volume of propellable matter to be provided into the propellable matter reservoir 40. Exemplary propellable matter regulating means include valves and pumps.

As previously indicated, exemplary embodiments of the source of propellable matter 20 are set forth in Figures 4A and 4B. These embodiments depict sources of propelled material which are capable of having a selected volume of propellable matter discharged therefrom.

In one embodiment, as seen in Figure 4A, the source of propellable matter comprises (1) propellable matter supply means 21 in the form of a syringe 21a having an outlet, and (2) a propellable matter supply line 23 having an inlet and an outlet. The outlet of the syringe 21a is in selective fluid communication with the inlet of the propellable matter supply line 23. The outlet of the propellable matter supply line 23 is in turn in fluid communication with an inlet of multipurpose valve 70 leading to the propellable matter reservoir 40.

In another embodiment, as seen in Figure 4B, the source of propellable matter comprises (1) propellable matter supply means 21 in the form of a propellable matter supply tank 21b having an outlet (2) a propellable matter regulating means 22 in the form of a propellable matter valve 22a, and (3) a propellable matter supply line 23 having an inlet and an outlet. The outlet of the tank 21b is in selective fluid communication, via propellable matter valve 22, with the inlet of the propellable matter supply line. The outlet of the propellable matter supply line 23 is in turn in communication with an inlet of valve 70 leading to the propellable matter reservoir 40.

Depending upon the time that the propellable matter is maintained in the propellable matter supply tank 21b, the propellable matter supply tank 21b may be in operative combination with a temperature control means 25 as shown in the alternate embodiment of Figure 4B to maintain the propellable matter at a desired temperature. Exemplary temperature control means include thermocouples implanted in a block heater surrounding the propellable matter supply tank 21b.

Depending upon the relative viscosity of the carrier medium, the propellable matter supply tank 21b may be in operative combination with an agitation means 24, to maintain the particles in suspension in the carrier medium. Exemplary agitation means include reciprocating syringe pumps, magnetic stir bar agitation, valving arrangements and pumps to create alternating directions of flow within the propellable matter supply tank 21b. Preferably, the agitation means will be selected so as also to provide alternating directions of flow within the propellable matter reservoir 40, e.g. a reciprocating syringe pump.

As seen in Figures 1 and 2, the outlet of the propellable matter reservoir 40 may be in selective communication, via multipurpose valve 70, with the inlet of recovery means 60.

The recovery means 60 allows for overflow of excessive propellable matter from the propellable matter reservoir 40. Recovery means 60 includes any means for capturing propellable matter. A preferred recovery means comprises a tube which is sealed from which material may be drawn out. Such a design also permits an open system to which an agitation means, e.g., a reciprocating syringe pump, may be selectively combined to provide alternating directions of flow within the propellable matter reservoir 40.

The recovery means 60 may be fabricated of any material which is capable of containing and not having a deleterious effect upon the propellable matter. Preferably, to avoid overflow of significant quantities of suitable propellable matter from the propellable matter reservoir, the recovery means 60 may be made of a transparent material to allow easy measurement of the material contained therein. Exemplary materials capable of visual inspection of overflow include polytetraflouroethylene (PTFE).

### Delivery Means

As seen in Figures 1, 2, and 7 the outlet of the propellable matter reservoir 40 may be in selective communication, via multipurpose valve 70, with the inlet of delivery means 50.

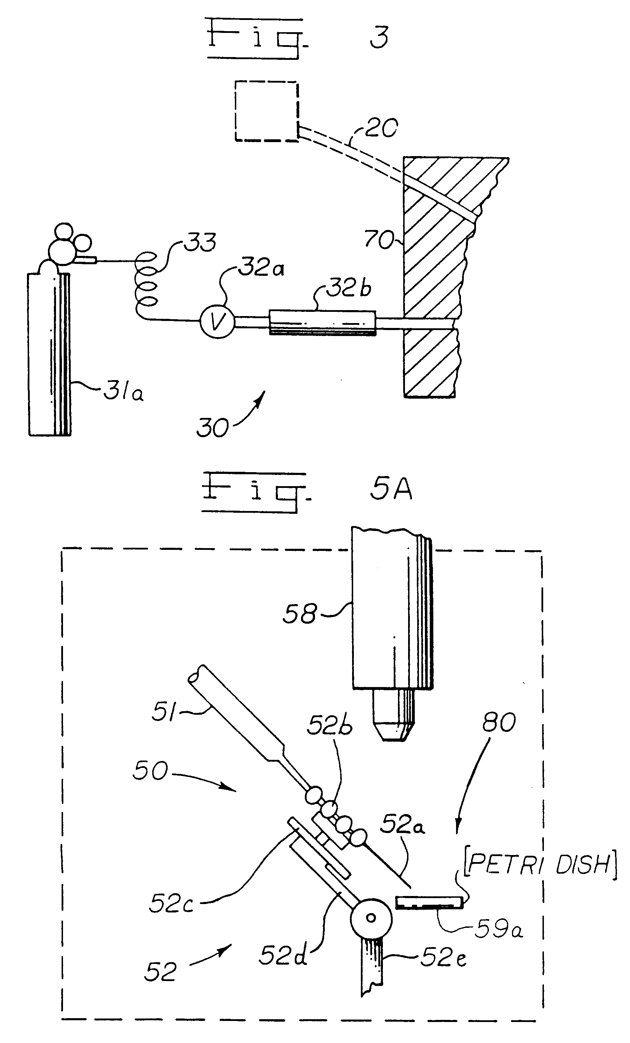

The delivery means 50 particularly shown in Figures 5A and 5B provides relatively precise aiming of the propellable matter. The delivery means 50 may comprise a macroaiming means 51 and/or a microaiming means 52. Because of the force of the propellable matter exiting from the propellable matter reservoir 40 (shown in Figures 1 and 2), it is preferred that when the delivery means 50 comprises microaiming means 52, it also comprises macroaiming means.

As seen in Figure 5A, the macroaiming means 51 in turn is in fluid communication with microaiming means 52.

Generally, the macroaiming means 51 is selected to have an inner surface which defines a bore of a length, geometry and diameter sufficient to prevent substantial loss of acceleration of the propellable matter due to gas bypassing the propellable matter. Preferably, the macroaiming means 51 is a tube selected to have an inner surface defining a generally cylindrical bore with an inner diameter of between 500 microns and 2000 microns, preferably 750 to about 1250 microns.

The macroaiming means 51 may be made of any material capable of maintaining its shape under the conditions of delivery. Exemplary materials include glass, plastic and stainless steel.

When the delivery means consists of only the macroaiming means 51, it may be used to accelerate propellable matter in a so-called "shotgun" firing pattern. When the delivery means comprises microaiming means 52, it allows relatively more precise aiming of the propellable matter.

In a first embodiment, as seen in Figure 5A, the microaiming means 52 comprises a pipette 52a, which is in fluid communication with the macroaiming means 51; a microinstrument 52b placed in a holder 52c rigidly connected to a mobile tool 52d of a three-way micromanipulator 52e. The micromanipulator 52e may comprise a joystick (not shown) that controls the position of pipette 52a.

The pipette 52a should be selected to have an internal diameter of sufficient lumen size to pass propellable matter. The internal diameter of the pipette is generally between about 10 microns and about 500 microns, preferably between about 100 to about 250 microns. Pipettes are conventionally prepared from capillary tubing according to well-known techniques (see generally, Graessman, *et al*. (1980), In: Methods in Enzymology, 65:816-825).

Exemplary microaiming means 52 may beneficially be constructed from conventional microinjection devices, which, given the teachings provided herein, may be relatively easily modified to define microaiming means by a skilled artisan.

Conventional microinjection devices and techniques are taught in the following references: United States Patent 4,743,548; Yamamoto, et al. (1982), Exp. Cell Res., **142**:79-84; Purres (1981),*Methods for Intracellular Recording and Ionophoresis*; Ocho, et al., (1981), Acta Med. Okayama, **35**:381-384; Lawrence and Davies (1985), Plant Cell Rep., **4**: 33-35; Steinbiss, et al. (1984) In: Proceedings of the 1984 Wye International Symposium, Experimental Manipulation of Ovule Tissue: Their Micromanipulation, Tissue Culture and Physiology; Steinbiss and Stabel (1983), Protoplasma, 116:223-227; Morikawa and Yamada (1981), Plant Cell Physiol., 26:229-236; Graessman, *et al*. (1980), In: Methods in Enzymology, 65:816-825; and Lin and Ruddle (1981), Exp. Cell Res., 134:485-488.

Further, while capable of being visually sighted, the delivery means preferably comprises a sighting means 58. The sighting means should provide a magnification effective to focus the target matter, preferably between 10X and 200X. Exemplary sighting means include dissecting microscopes and boroscopes.

In a second embodiment, as seen in Figure 5B, the microaiming means 52 comprises pipette 52a in operative combination with sighting means 58.

As seen in Figures 1, 5A, 5B, and 7, the outlet of the delivery means opens into a propulsion zone 80, into which the target matter may be placed.

The target matter may be placed on a suitable platform. For example, a petri dish 59a may be used to contain the target matter. To assist in the aiming of the delivery means 51, the target matter may preferably be placed on an X-Y translational stage 59b.

Preferably, the propulsion zone 80 is environmentally controlled. Generally, the propulsion zone may be defined by a chamber.

For example, a vacuum may be drawn in the propulsion zone. The degree of vacuum should be sufficient to avoid decreasing the velocity of the particles due to the reduction caused by air resistance, and to prevent scattering of the propellable matter. Preferably, the degree of vacuum in the propulsion zone may vary from 0 atm. to 0.5 atm.

Suitable environmentally-controlled chamber is taught in "Fisher 88", Laboratory Equipment Catalog of Fisher Scientific, Inc., Pittsburgh, PA.

### Velocity Measuring Means

In another embodiment, the apparatus 10 comprises a velocity measuring means 90 as indicated in Figures 1, 6, and 7. Any device capable of monitoring the velocity of the propellable matter is suitable for use as the velocity measuring means.

Generally, the velocity measuring means may be positioned at any location from which the velocity of the accelerated propellable matter may be reproducibly measured. Preferably, velocity measuring means 90 may be positioned on delivery means 50.

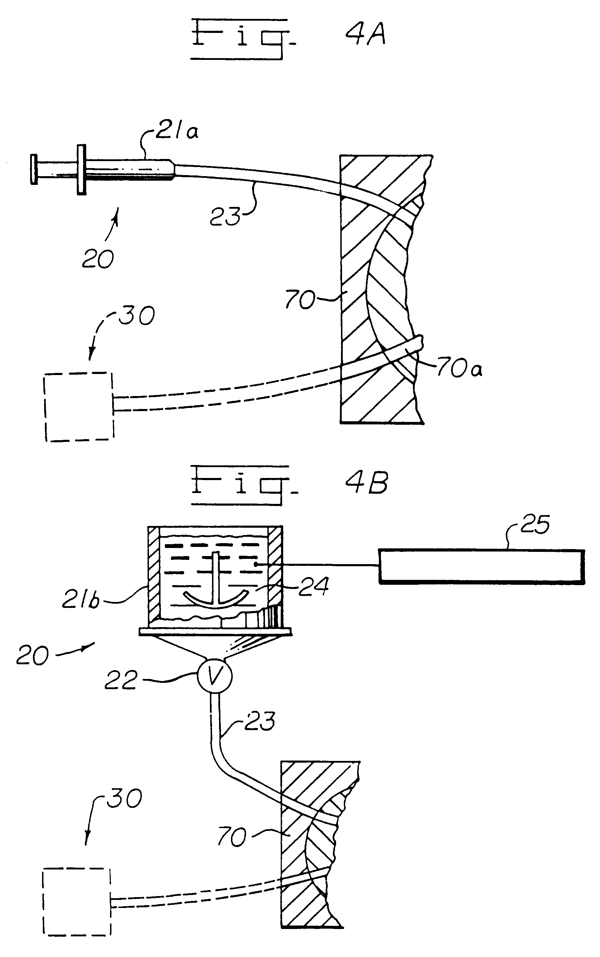

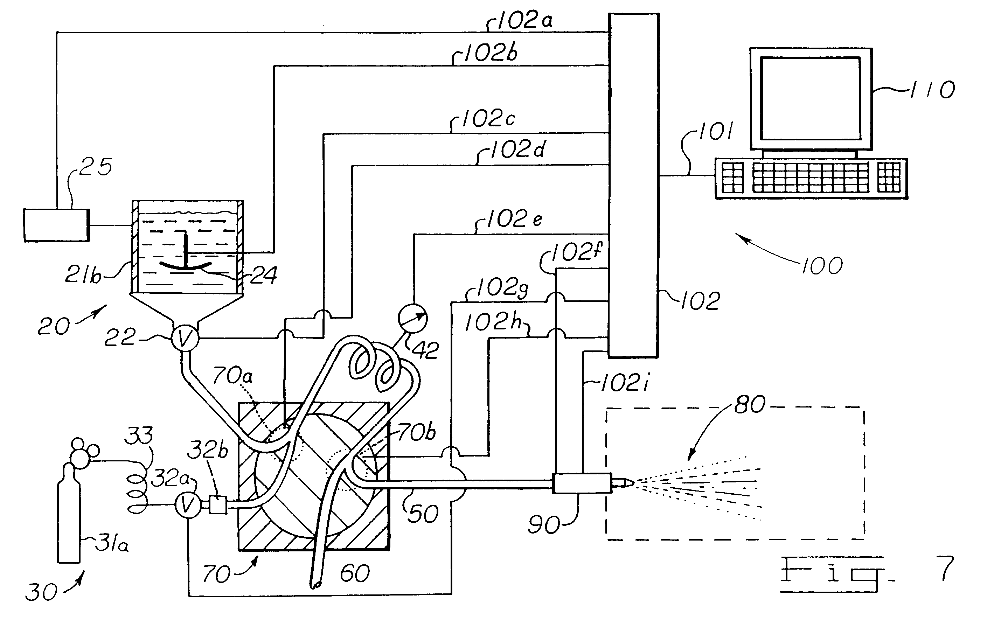

As seen in Figure 6, an exemplary velocity measuring means 90 comprises a first velocity sensor 92 and a second velocity sensor 94, said sensor being positioned at successive locations across delivery means 50.

The first velocity sensor 92 comprises source means 92a and sensor means 92b. The second velocity sensor 94 comprises source means 94a and sensor means 94b.

Exemplary source means 92a and 94a include infrared emitters, such as infrared light emitting diodes (LED), or any other similar light source. When the source mounting means comprises infrared emitters, the delivery means is beneficially constructed of a transparent material, such as glass or clear plastic.

Exemplary sensor means 92b and 94b include photodiodes, phototransistors or photovoltaic cells.

Generally, the first and second sensors may be in operative combination with the multipurpose valve 70 via an electric circuit.

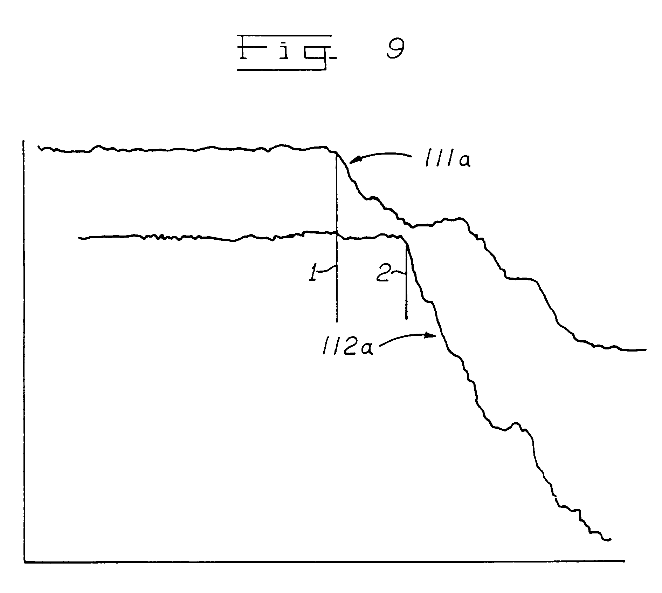

Generally, the circuit should provide for the measurement of the time of flight (as seen in the "Examples" section, below, and Figure 9) between first and second sensors. Preferably, the circuit may provide "sequence timing", to ensure that all parts of the velocity measuring means 90 are synchronized with the acceleration of propellable matter. After the circuit has ensured the timing status, the circuit may provide operative communication between the velocity measuring means 90 and multipurpose valve 70, whereby a preselected time interval of effective electric power to operate multipurpose valve 70 provides communication between the source of gas under pressure 30 and the propellable matter reservoir 40.

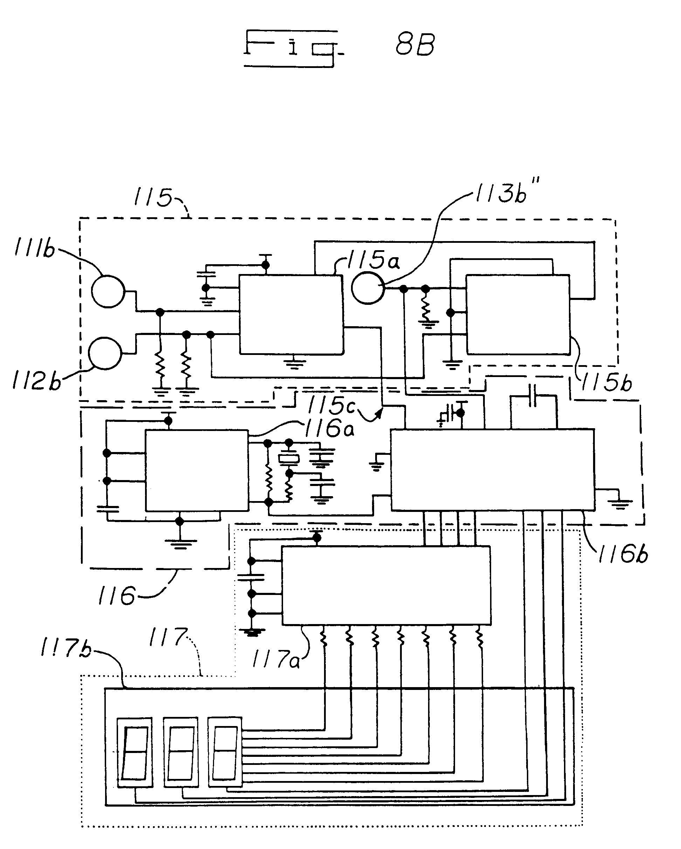

Any number of circuits may be devised by a skilled artisan to achieve the requirements set forth above. Exemplary circuits for the velocity detection means are shown in Figures 8A and 8B and will be discussed hereinafter.

### Synchronizing Means

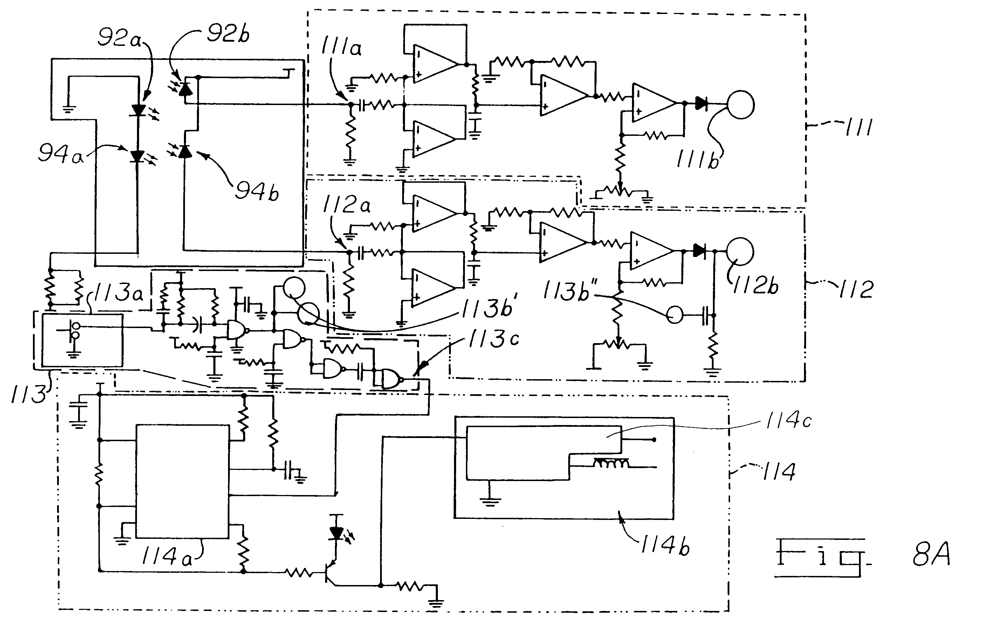

In another embodiment as seen in Figure 7, the apparatus 10 may be in operative combination with a means effective to synchronize at least some elements for loading and accelerating the propellable material. Synchronization of at least some apparatus elements is intended to reduce the number of steps that an operator would have to perform, consequently decreasing the time necessary between actuations and increasing the throughput.

An exemplary embodiment of synchronizing means is set forth in Figure 7. The synchronizing means 100 comprises a computer 110 being in operative combination with apparatus 10.

The computer 110 is in communication, via line 101, with box 102, having a series of sensor and command leads therefrom.

A first lead 102a may be in sensor and command communication with the temperature control means 25, whereby the temperature of the propellable matter supply tank 21b is automatically regulated.

A second lead 102b may be in sensor and command communication with the agitation means 24, whereby the propellable matter is agitated at a selected rate.

A third lead 102c is in command communication with the propellable matter regulating means 22. The lead maintains the source of propellable matter in an open or closed position.

A fourth lead 102d is in sensor communication with third lead 102c and command communication with multipurpose subvalve 70a. When the source of propellable matter is maintained in the open position, multipurpose valve 70 provides selective fluid communication between the source of propellable matter 20 and propellable matter reservoir 40, and selective fluid communication between the propellable matter reservoir 40 and the recovery means 60.

A fifth lead 102e is in sensor and command communication with the temperature control means 42, whereby the temperature of the propellable matter reservoir is regulated.

A sixth lead 102f is in sensor communication with the fourth lead 102d and command communication with the velocity detection means 90. When a selected volume of propellable matter is in the propellable matter reservoir 40, a timing cycle for the firing of apparatus 10 is initiated.

A seventh lead 102g is in sensor communication with sixth lead 102f and gas valve 32a. When the timing cycle of the velocity detection means is ensured, the gas regulating means is maintained in the open position.

An eighth lead 102h is in sensor communication with the seventh lead 102g and multipurpose subvalve 70b. When the source of gas under pressure is in the open position, the multipurpose valve 70 provides selective pneumatic communication between the source of gas under pressure 30, via the propellable matter reservoir 40, and the delivery means 50.

A ninth lead 102i is in sensor communication with the velocity detection means 90. After a selected volume of gas under pressure is emitted into the propellable matter reservoir 40 and an accelerated volume of propellable matter passes the velocity detection means 90, the velocity of the propellable matter is displayed.

Generally, the synchronizing means may be any process control system which is capable of assimilating data provided by the various sensor leads, and operating the apparatus via the various command leads. Exemplary synchronizing means include single board microcontrollers, or the CAMILE™ (Trademark of The Dow Chemical Company) data acquisition and process control system, commercially available from The Dow Chemical Company.

### Operation

The apparatus, as seen in Figure 1, may be operated as follows. Target matter may be selectively positioned in the propulsion zone at a predetermined distance from the delivery means.

When the propellable matter or target matter includes cellular biological material, the target matter will be selectively positioned at a distance effective to permit contact with the propellable matter which will not be lethal to a substantial number of the cellular biological material (though some cells may die).

Generally, the target matter will be placed at a distance of between 1 cm and 100 cm from the outlet of the delivery means.

When the delivery means comprises a macroaiming means only, i.e. without a microaiming means, the target matter will be placed at a distance of from 5 cm to 20 cm to the delivery means outlet. When the delivery means comprises a microaiming means, the target matter will be placed at a distance of 1 millimeter (mm) to 100 cm to the delivery means outlet.

The apparatus as generally set forth in Figure 1, and specifically embellished in selected Figures, may be operated as follows. As seen in Figures 1, 2 and 4B, multipurpose valve 70 and propellable matter regulating means 22a are set to provide fluid communication between the propellable matter supply means 21 and the propellable matter reservoir 40. Consequently, propellable matter is allowed to pass from the source of propellable matter 20 and into the propellable matter reservoir 40.

As seen in Figures 1 and 2, multipurpose valve 70 is also set to provide fluid communication between the propellable matter reservoir 40 and the recovery means 60. Any volume of propellable matter in excess of the volume of the propellable matter reservoir 40 is released into the recovery means 60.

As seen in Figures 1, 2, and 3, multipurpose valve 70 and gas regulating means 32a are then set to provide pneumatic communication between the gas supply means 31 and the propellable matter reservoir 40. Consequently, a selected volume of gas under sufficient pressure to accelerate the propellable matter at a desired velocity is allowed to pass from the source of gas under pressure 30 into the propellable matter reservoir 40.

Multipurpose valve 70 is also set to provide pneumatic communication between the propellable matter reservoir 40 and the delivery means 50. Once the gas is discharged into the propellable matter reservoir 40 it contacts against the propellable matter, which is accelerated into delivery mean 50, if present.

As seen in Figures 1 and 6, while in the delivery mean 50 the propellable matter will pass first sensor 92 and second sensor 94. First and second sensors 92 and 94 provide means to record the velocity of the propellable matter, permitting reproducible acceleration of the propellable matter.

Preferably, the propellable matter will be accelerated to an effective velocity. The "effective" velocity will vary depending upon the desired results.

Generally, for purposes of introducing biological material into cellular biological material, the velocity of the propellable matter should be effective to cause the biological material to penetrate the cell membrane, and wall if present. Preferably for such systems, the propellable matter should have a velocity at the point of exiting the delivery means of between 200 miles per hour (mph) to 1200 mph (89.4 to 536.4 m s⁻¹).

As should be apparent, the velocity of the propellable matter is dependent upon a variety of known parameters. Such parameters include, but are not limited to, the length and inner diameter of the delivery means; the gas pressure; the flow properties of the gas; the flow properties of the propellable matter; the physical properties of the propellable matter; the distance between the outlet of the delivery means and the target matter; and the volume and flow properties of the medium in which the target matter is cultured.

The flow characteristics of the propellable matter are not particularly critical. Thus, the propellable matter may be accelerated in plug flow, or turbulent flow. By "plug flow" it is meant that the propellable matter moves as a generally continuous mass, and the gas and propellable matter zones will not substantially intermix. By "turbulent flow" is meant fluid flow in which the velocity at a given point varies erratically in magnitude and direction, and consequently some mixing of the gas and propellable matter occurs. If turbulent flow occurs, the propellable matter and gas will interact to create a dispersion and consequently provide a broader columnar beam of propellable matter when discharged from the apparatus.

Because the gas under pressure contacts the propellable matter directly and is itself released into the propulsion zone, the propellable matter loaded into the reservoir is reproducibly discharged into the propulsion zone. A minor but reproducible proportion of propellable matter remains in the delivery means. Basically, the only propellable matter which is not discharged into the propulsion zone is that which adheres or wets to the inner walls of the valves and the discharge means. Such propellable matter is referred to as "hang up". The quantity of the hang up will depend upon and vary with the viscosity of the particular propellable matter.

The velocity of the propellable matter is measured as it passes the velocity detection means. More specifically, the time of flight between the first and second sensors of velocity detection means, located on the delivery means, indicates the velocity of the propellable matter.

By providing reproducible measurements of the time of flight of the propellable matter, one of ordinary skill in the art may adjust the various parameters which affect the velocity of the propellable matter. By adjusting the parameters, the skilled artisan may select the optimum velocity of the propellable matter.

A desired volume of propellable matter exits from the delivery means into the propulsion zone.

The operation is completed by selectively setting the multipurpose valve 70 in order to block the flow of gas from the gas reservoir 32 into the propellable matter reservoir.

The previous discussion is intended to provide a general idea of the criteria to be considered in the operation of the apparatus according to the present invention. Given the teachings above, the selection of operating conditions for each system employed will be obvious to one of ordinary skill in the art.

The present invention may be used in various biological sciences, including transformation of plant cells, animal cells, and microorganisms.

An appealing feature of the present invention is that it allows treatment of plant cells whose walls are intact. Because the obstacle of regenerating whole plants from protoplasts may be circumvented, the genetic engineering of important grain species may be facilitated.

Two important targets for plant germline transformation are pollen or eggs, and meristem domes or tissue culture cells (from intact plants and embryos, or from tissue culture). Transformation of pollen, eggs, or meristem domes are possible methods for sexually-propagated crops, while transformation of tissue culture cells or meristematic domes are possible methods for asexually-propagated crops. Each of these approaches is capable of producing transformed whole plants.

Tissue culture cells may be transformed, and then cultured to provide somatic embryos or meristem domes, which in turn regenerate into transferred plants.

Meristem transformation, for example, may be achieved by surgically exposing the meristematic dome, and bombarding it with DNA-bearing particles, permitting a large number of meristematic cells to be transformed. The transformed meristems are capable of being grown into chimeric shoots, from which stable transformed sectors are selected.

For a general discussion of a process wherein genes were inserted into immature embryo axes, which are then used to regenerate plants, see McCabe, *et al*. (1988), Bio/Technology, **6**:923-926.

Finally, chloroplast transformation in *Chlamydomonas*, a unicellular algae, using micro-projectile bombardment has also been reported (Boynton, *et al*. (1988), Sci., **240**:1543-1537. Three mutants of the chloroplast *atpB* gene of the *Chlamydomonas reinhardtii* were transformed with chloroplast DNA containing the wild-type gene. Photosynthetic capacity was restored in the transformants.

The genetic transformation of small groups of cells in animal tissues is now possible using the method and apparatus of the present invention. Such a therapy provides a non-infectious, but highly efficient, mechanism for the transformation of animal tissues, *in situ*. For a general discussion, see Sanford, *et al*., *supra*.

The transformation of mitochondria in yeast by bombardment with projectiles has been reported (Johnston, *et al*. (1988), Sci., **240**: 1538-1541. The reference teaches the transformation of a nonreverting strain of yeast which is respiratory deficient because of a deletion in the mitochondrial *oxi* 3 gene with DNA sequences that could correct the *oxi* 3 deletion. Respiratory-competent transformants were obtained which contained a homologous replacement of the defective *oxi* 3 gene.

The fate of foreign biopolymers such as RNA, protein, lipids and both organic and inorganic chemicals may be analysed by shooting particles coated with these molecules or combinations of molecules.

Additionally, isolated subcellular organelles such as mitochondria or chloroplasts may be transformed and then reinsertion of the transformed organelles into plant cells or protoplasts.

### Examples

The following examples are presented to further illustrate but not limit the scope of this invention. All parts and percentages are by weight unless otherwise indicated.

The propellable matter was prepared as follows.

Carrier particles coated with DNA-containing plasmids were suspended in a carrier medium. More specifically, the plasmid was adsorbed to the surface of gold particles. The plasmid contained a gene which encodes for the enzyme beta-glucuronidase (GUS gene) and which was under the control of a 35s California Mosaic Virus (CaMV) promoter (gene, promoter and regulatory sequences obtained from Clontech Laboratories, Inc., Palo Alto, California, USA). The gold particles were spherical powder of 1.5 to 3.0 microns in diameter (commercially available from Alfa Products, Danvers, MA).

To accomplish adsorption, 50 »l of a plasmid solution (1.8 microgram (»g) of DNA per microliter (»l) of 0.01 molar (M) Tris buffer, pH 8.0, with 0.001 M ethylene diamine tetraacetic acid (EDTA)) was added to 400 »l of a suspension of gold carrier particles (300 milligrams (mg) of gold carrier particles per milliliter (ml) of distilled water). The DNA was precipitated by the addition of 74 »l of a 2.5 M calcium chloride solution and 30 »l of a 0.1 M spermidine solution. The coated carrier particleswere allowed to settle to the bottom of an Eppendorf tube and the resultant clear liquid was completely drawn off. The carrier particles were resuspended in 500 »l ethanol (100%) (carrier medium). One carrier particle was coated with approximately 10 copies of the plasmid.

The target matter was prepared as follows. Suspension cultures of cultivar Black Mexican Sweet (BMS) pea cells were obtained from Professor Virginia Walbot (Stanford University). The BMS are described in Sheridan (1975), J. Cell Biol., **67**:3969. These cultures were routinely maintained in a liquid Murashige and Skoog (MS) medium (Physiol. Plantarum, 1962, 15:473-496) supplemented with 2,4-dichlorophenoxyacetic acid (2 mg/liter).

In preparation for bombardment with the carrier particles, a 100 mg sample of cells was collected from suspension on a 7 cm Whatman no. 1 filter paper by vacuum filtration on a Buchner funnel. The filter paper with the cells were placed in a 9 cm Petri dish 59a which contains the MS medium, described above, in solid form.

The apparatus used to accelerate the coated gold particles at the BMS pea cells consisted of the following elements, which are previously described in figures 1, 2, 3 and 4A:
(1). A 1A size gas cylinder (i.e., gas supply means 31) filled with helium gas was in fluid communication with a stainless steel capillary tube having dimensions of 7′ x 0.02˝ I.D. (i.e., gas supply line 33). The gas supply linewas in operative combination with a standard two-stage pressure regulating valve, commercially available from Victor Equipment Co. (i.e., gas regulating valve 32a). The gas supply line was in turn in communication with a high pressure stainless steel gas chamber having dimensions of 1/8˝ O.D. x 3˝ length (ie., gas reservoir 32b).
(2). A 3 cubic centimeter (cc) Luer Lok sterile syringe (i.e., propellable matter supply means 21a) was in fluid communication with 1/10˝ Teflon (trademark of E. I. DuPont de Nemours Co., Wilmington, DE) FEP tubing (i.e., propellable matter supply line 23). The tube iwas in operative combination with a Rheodyne Model 7030 3-way valve (i.e., multipurpose valve 70).
(3). A clear 1/10˝ OD FEP tube (i.e., recovery means, 60).
(4). A pyrex glass tube having dimensions of 10 cm x 1.2 mm O.D. x 0.8 I.D. mm (ie., delivery means 50).
(5). A velocity detection means comprised two sensors adjacently positioned relative to each other on the delivery means 50. Each sensor (i.e., first sensor 92 and second sensor 94) comprised a photodiode and an infrared emitter. The photodiodes are commercially available from Motorola Semiconductor Products, Inc. (Phoenix, AZ, USA) under the trade designation MRD5ØØ, and the infrared emitters are commercially available from General Electric under the trade designation LED55C.
   The first and second sensors were in operative combination via an electric circuit having a design as shown in the schematic representation of Figures 8A and 8B.
   Provided below is a detailed explanation of Figures 8A and 8B, depicting the operative combination between the velocity detection means 90 and the multipurpose valve 70.
   Block 113 provided sequence timing to ensure that after actuation means (i.e., firing button 113a) is pressed, all other parts of the circuit were prepared for the timing cycle. Block 113 sent signal 113b′ simultaneously indirectly, via block 112 (received as signal 113b''), to flip flop 115a (received as signal 112b) to stop the timer, and signal 113b′ directly to flip flop 115b (received as signal 113b'') to reset block 115 to accept only one start signal (111b) and to clear the count timer block 116 to zero. The indirect signal is provided as a fail safe to stop the timer in case the optics of second sensor 94 (as depicted by source means 94a and sensor means 94b) did not register the passing of propellable material. Block 113 then provided signal 113c to begin the actuation of a timer (i.e., 114a on Figure 8A) in block 114.
   Block 114 accepted signal 113c from block 113 and then provided a preselected time interval via a solid state relay (i.e., 114c on Figure 8A), operating a 4-way valve (i.e., 114b on Figure 8A) that sent a selected volume of air pressure to a pneumatic actuator (not shown) on the multipurpose valve 70. This operated valve 70 to provide communication between the source of gas under pressure and the propellable matter reservoir.
   Block 111 accepted signal 111a, generated by the propellable matter passing between the source means and sensor means of first velocity sensor 92 (as depicted by source means 92a and sensor means 92b) and conditioned the signal and produced a sharp rising voltage signal corresponding in time to the decreasing voltage of signal 111a, i.e., a "start" signal 111b capable of starting a timer (block 116 in Figure 8B).
   Block 112 accepted signal 112a, generated by the propellable matter passing between combination of source means and sensor means of second velocity sensor 94, and produced a sharp rising voltage signal corresponding in time to the decreasing voltage of signal 112a, i.e. a "stop" signal 112b capable of stopping the timer (block 116 in Figure 8B).
   Flip flop 115a accepted the "start" signal 111b from block 111, the "stop" signal 112b from block 112 and flip flop 115b accepted the "clear timer" signal (113b'') from the sequence block 113, and maintained the timing status, i.e., allowing only one timing cycle per delivery cycle of the propellable matter. Flip flop 115a generateed signal 115c, which is sent to block 116.
   Block 116 compriseed a quartz crystal stabilized timer comprising a clock integrated circuit 116a and a digital counter 116b. Block 117 compriseed a multiplexed binary coded decimal (BCD) to seven segment display decoder (i.e., 117a on Figure 8B) and an LED display (i.e., 117b on Figure 8B). Block 117 thus displays the time of flight of the most recent propellable matter.
   Digital counter 116b accepted the timing status signals 113b'' and 115c from block 115. These timing status signals were "timer run" (i.e., 115c) and "timer clear" (i.e., 113b''). When the timer run signal was. 0̸ volts, the timer block counts micro-seconds up from zero. When the timer run signal was. 12 volts, the timer stopped and held the last count. When the timer clear signal was 0̸ volts, the timer ran. When the timer clear signal was 12 volts, the timer was cleared to zero. The display block 117 accepted BCD numeric signals from block 116, and thus displayed the most recent time of flight of the propelled material in micro-seconds on the front panel display.
   Although an exemplary circuit has been shown, many circuits were possible to achieve the same result.
(6) A dual three-way valve (multipurpose valve 70), having two subvalves (subvalve 70a and 70b), commercially available from Rheodyne, Inc., Cotati, CA under the trade designation model 7030 ARV. The subvalves of the dual three-way valve 70 were in operative combination with a pnuematic actuator, kit #41687 commercially available from Anspec Co., Ann Arbor, MI. The pnuematic actuator was driven by air supplied from a four-way solenoid valve, commercially available from the Automatic Switch Company (ASCO), Florham Park, NJ.
(7). An external loop 40 made of stainless steel, 1/16˝ O.D. (i.e., propellable matter reservoir) was in operative combination with the first and second three-port valves.

The first three-port valve provideed selective fluid communication between the propellable matter supply means or the gas reservoir, and the propellable matter reservoir.

The second three-port valve provideed selective fluid communication between the propellable matter reservoir and the recovery means or the delivery means.

The multipurpose valve was initially set in order to provide fluid communication between the syringe and the propellable matter and to block pneumatic communication between the gas reservoir and the propellable matter reservoir.

The syringe, which was capable of delivering 1 ml of the propellable matter (carrier medium suspension of coated particles), was placed in fluid communication with the propellable matter supply line. The propellable matter supply line was in fluid communication with the propellable matter reservoir.

The propellable matter regulating valve 22a was opened to provide fluid communication between the propellable matter supply means and the propellable matter reservoir. After a selected volume of propellable matter was emitted into the propellable material reservoir, the propellable matter valve was closed.

Any volume of propellable matter in excess to the volume of the propellable matter reservoir was released through the recovery means.

The gas regulating valve 32a was opened to provide pneumatic communication between the gas supply means and the gas reservoir. A gas pressure of about 1000 psi was obtained in the gas reservoir. The gas regulating valve was then closed.

The remainder of the operation was controlled via the circuit set forth in Figures 8A and 8B. The circuit operated as follows.

After the firing button 113a was pressed, block 113 provided a sequence timing of 0.05 seconds to halt the digital timer of block 116. Block 115 held the status of block 116, i.e., if by error the digital timer was still timing after a previous actuation of the apparatus, the digital timer was cleared to zero in preparation for the next timing cycle. After the digital timer was stopped, the timer status of block 115 was set to accept only one start signal from block 111.

Next, the interval timer block 114 was started. The interval timer block 114 supplied a preset time interval of 0.8 seconds of 120 volts alternating current (VAC) to actuate valve 114b, supplying 65 to 75 pounds per square gauge (psig) of air to drive a pneumatic actuator (not shown) on the multipurpose valve 70 to set the multipurpose valve to provide selective communication between the source of gas and the propellable matter reservoir, which resulted in the acceleration of the pressurized gas and propellable matter past the first and second sensors.

As the leading edge of the propellable matter passed the first sensor of the velocity detection means, an electrical signal (111a) was generated. When conditioned by block 111, the electric signal was the signal used by block 115 to start the digital timer block 116.

Similarly, as the leading edge of the propellable matter passed the second sensor of the velocity detection means, an electrical signal (112a) was generated. When conditioned by block 112, the electric signal was the signal used by block 115 to stop the digital timer block 116. The time of flight of the leading edge of the propellable matter between the first sensor means and the second sensor means was displayed on (the 3 digit, 7 segment), LED display (i.e., 117b) of block 117 a multisegment LED indicator.

Figure 9 sets forth the raw data obtained from the operation of the apparatus. Electric signal 111a was derived from sensor 92 and electric signal 112a was derived from sensor 94. The sensors were separated by 2.8 centimeters. As the propellable matter passes between either sensor the electric signal will shift from a baseline value. For electric signal 111a this shift is shown at the point of line 1, and for electric signal 112a this shift is shown at the point of line 2. The time of travel between the first and second sensors, as shown as the distance between lines 1 and 2, was 60 microseconds. This indicates that the propellable matter was traveling at a velocity of about 1044 miles per hour (6051 meters/sec).

The propellable matter exited from the delivery means toward the BMS cells in a propulsion zone. The propulsion zone was defined by a vacuumed chamber, the design of which was taught in "Fisher 88", *supra*, p. 114. The pressure in the propulsion zone was about 0.1 atm absolute.

The operation was completed by selectively setting the valve in order to block the passage of gas from the gas reservoir through the valve into the delivery means, and to provide fluid communication between the syringe and the propellable matter overflow reservoir.

Monolayers of BMS cells on Petri dishes (i.e., Petri dish 59a) of MS medium were bombarded simultaneously. There were 100 mg BMS cells on each Petri dish. The carrier particles coated with the GUS-containing plasmid were delivered into the BMS cells.

Following bombardment of the BMS cells, the culture was incubated in the dark for 2 days at 27°C. After two days, the cells were assayed for GUS activity.

Expression of the GUS gene in the BMS cells was observed by using the GUS histochemical assay (5-Br-4-Cl-3 indolyl-beta-D-glucuronic acid [X-gluc] substrate and procedure obtained from Clontech Laboratories, Inc., Palo Alto, CA). The BMS cells and cell clumps were incubated at 37°C for 24 to 48 hours in solution of 1 millimolar (mM) X-gluc containing 0.5 mM potassium ferricyanide, 0.5 mM potassium ferrocyanide and 10 mM EDTA. The cells and cell clumps which turn blue with this assay were scored as positive for GUS activity.

The results are set forth in Table 1.

**TABLE 1**

| Bombardment Schedule | Number of BMS cells or cell clumps with GUS activity/petri dish |
|---|---|
| cells bombarded with gold particles coated with plasmid containing GUS gene | 4 |
| repeat | 5 |
| repeat | 6 |
| repeat | 4 |
| repeat | 5 |
| repeat | 2 |
| repeat | 10 |
| repeat | 8 |
| repeat | 9 |

These findings indicate that particle bombardment was used to deliver DNA into intact plant cells simultaneously and that the gene introduced by this process can subsequently be expressed.

As is apparent from the foregoing specification, various modifications of the invention in addition to those shown and described herein will become apparent to those skilled in the art from the foregoing description and accompanying drawings. For this reason, it is to be fully understood that all of the foregoing is intended to be merely illustrative and is not to be construed or interpreted as being restrictive or otherwise limiting of the present invention, excepting as it is set forth and defined in the hereto-appended claims.

## Claims

1. An apparatus comprising the following:
(a) a source of gas under pressure (30) having a gas outlet and a source of propellable matter having an outlet;
(b) a propellable matter reservoir (40) having an inlet and an outlet, characterized in that it further comprises:
(c) a multipurpose valve (70), wherein the multipurpose valve (70) is of a design providing selective communication between the outlet of the source of gas under pressure (30) that has a gas supply means (31) having an outlet and a gas regulating means (32) being in operative combination with the gas supply means (31) to provide selective communication with the gas supply means (31) outlet and the inlet of the propellable matter reservoir (40), or the outlet of the source of propellable matter (20) that has a propellable matter supply means (21) to provide selective communication between the propellable matter supply means (21) outlet, and a propellable matter regulating means (22) being operative in combination with the propellable matter supply means (21) outlet and the inlet of the propellable matter reservoir (40).

2. The apparatus of Claim 1, wherein the apparatus further comprises:
(e) a recovery means (60) having an inlet, and
the multipurpose valve (70) is of a design providing selective communication between either the outlet of the source of gas under pressure (30) or the outlet of the source of propellable matter (20), and the inlet of the propellable matter reservoir (40), and providing selective communication between either the inlet of the delivery means (50) or the inlet of the recovery means (60), and the outlet of the propellable matter reservoir (40).

3. The apparatus of Claim 2, wherein the apparatus further comprises:
(f) a delivery means (50) having an inlet and an outlet, wherein the inlet of the delivery means (50) is in fluid communication with the outlet of the propellable matter reservoir (40), and
the multipurpose valve (70) is of a design providing selective communication between either the outlet of the source of gas under pressure (30) or the outlet of the source of propellable matter (20), and the inlet of the propellable matter reservoir (40), and providing selective communication between either the inlet of the delivery means (50) or the inlet of the recovery means (60), and the outlet of the propellable matter reservoir (40).

4. The apparatus of Claim 1, wherein the gas regulating means (32) comprises a gas valve (32a) and a gas reservoir (32b) having an inlet, where the gas valve (32a) provides selective pneumatic communication between the gas supply means (31) outlet and gas reservoir (32b) inlet.

5. The apparatus of Claim 1, further comprising a temperature regulating means (42), said temperature regulating means (42) being in operative combination with the propellable matter reservoir (40).

6. The apparatus of Claim 1, wherein the source of propellable matter (20) further comprises an agitation means (24), said agitation means (24) being in operative combination with the propellable matter supply means (21).

7. The apparatus of Claim 3, wherein the delivery means (50) comprises a macroaiming means (51).

8. The apparatus of Claim 3, wherein the delivery means (50) comprises a microaiming means (52).

9. The apparatus of Claim 3, wherein the apparatus comprises a velocity detection means (90).

10. The apparatus of Claim 9, wherein the apparatus comprises a first sensor (92) and a second sensor (94).

11. The apparatus of Claim 1, wherein the propellable matter reservoir (40) is in fluid communication with an environmentally-controlled chamber, said chamber defining a propulsion zone (80).

12. A method for introducing biological material into living cells, said method comprising the following steps:
(a) providing a predetermined volume of gas having a selected gas pressure;
(b) providing a predetermined quantity of a propellable matter, wherein the propellable matter is biological material suspended in a carrier medium; and
(c) contacting said propellable matter with the predetermined volume of a gas, wherein the propellable matter is accelerated at a selected target.

13. A method for introducing biological material into target matter, said method comprising the following steps:
(a) providing a predetermined volume of gas in a gas reservoir at predetermined pressures;
(b) providing, in a propellable matter reservoir, a predetermined quantity of a propellable matter, which comprises a suspension of a biological material in a carrier medium;
(c) contacting said propellable matter with the predetermined volume of a gas; and
(d) accelerating the propellable matter through a delivery means at the target matter.

14. The method of claim 12, wherein the propellable matter is accelerated at a selected target through a delivery means (50) and exits the delivery means (50) at a velocity of between 322 and 1931 km per hour (200 and 1200 miles per hour), 89.4 and 536.4 m s⁻¹

15. An apparatus comprising the following:
(a) a propellable matter reservoir (40) having an inlet and an outlet;
(b) a delivery means (50) having an inlet and an outlet, the inlet of the delivery means (50) being in communication with the outlet of the propellable matter reservoir (40); and
(c) a multipurpose valve (70) comprising a first subvalve (70a) having an outlet and a second subvalve (70b) having an inlet and an outlet, the outlet of the first subvalve (70a) providing selective communication with the inlet of the propellable matter reservoir (40) and the second subvalve (70b) providing selective communication between the outlet of the propellable matter reservoir (40) and the inlet of the delivery means (50).

16. The apparatus of claim 15, further comprising:
(d) a source of gas under pressure (30) having a gas outlet,
the first subvalve (70a) of the multipurpose valve (70) being capable of providing selective pneumatic communication between the outlet of the source of gas under pressure (30) and the inlet of the propellable matter reservoir (40),
the source of gas under pressure (30), when in communication with the propellable matter reservoir (40), discharges gas into the propellable matter reservoir (40) and under sufficient pressure to cause a propellable matter to exit the delivery means (50), when the propellable matter reservoir (40) is in communication with the delivery means (50), at a velocity effective to cause a noncellular biological material to enter a biological cell.

17. The apparatus of claim 16, wherein the source of gas under pressure (30), when in communication with the propellable matter reservoir (40), discharges gas under sufficient pressure to cause a propellable matter to exit the delivery means (50), when the propellable matter reservoir (40) is in communication with the delivery means, at a velocity of between 322 and 1931 km per hour (200 and 1200 miles per hour), 89.4 and 536.4 m s⁻¹.

18. The apparatus of claim 15, further comprising:
(e) a source of propellable matter (20) having an outlet, the first subvalve (70a) of the multipurpose valve (70) providing selective communication between the outlet of the source of propellable matter (20) and the inlet of the propellable matter reservoir (40).

19. A process for introducing biological material into target matter, said method comprising the following steps:
(a) providing an apparatus having
(i) a source of gas under pressure (30) having a gas outlet and a source of propellable matter having an outlet;
(ii) a propellable matter reservoir (40) having an inlet and an outlet;
(iii) a delivery means (50) having an inlet and an outlet, wherein the inlet of the delivery means is in communication with the outlet of the propellable matter reservoir; and
(iv) a multipurpose valve (70), wherein the multipurpose valve (70) is of a design providing selective communication between the outlet of the source of gas under pressure (30) and the inlet of the propellable matter reservoir (40); and
(b) positioning the target matter at a selected effective distance from the outlet of the delivery means; and
(c) aiming the delivery means at the target matter; and
(d) setting the multipurpose valve to provide fluid communication between the propellable matter supply means and the propellable matter reservoir, whereby a selected volume of propellable matter enters the propellable matter reservoir; and
(e) setting the multipurpose valve to provide pneumatic communication between the outlet of the gas supply means and the inlet of the propellable matter reservoir and the outlet of the propellable matter reservoir and the inlet of the delivery means, whereby a selected volume of gas contacts the propellable matter in the propellable matter reservoir.

20. The process of claim 19, wherein the velocity of the propellable matter after exiting the propellable matter reservoir is monitored by a velocity measuring means.

21. The process of Claim 20, comprising a step (f) of selectively setting the valve in order to block the passage of gas from the gas reservoir through the valve into the delivery means and to provide fluid communication between a syringe and a propellable matter overflow reservoir.

## Patentansprüche

1. Vorrichtung welche Folgendes umfaßt:
(a) eine unter Druck stehende Gasquelle (30) mit einer Gas-Austrittsöffnung und eine Quelle für vorwärts treibbares Material mit einer Austrittsöffnung;
(b) ein Vorratsbehälter für vorwärts treibbares Material (40) mit einer Eintrittsöffnung und einer Austrittsöffnung, dadurch gekennzeichnet, daß sie weiter umfaßt:
(c) ein Mehrzweckventil (70), worin das Mehrzweckventil (70) so ausgelegt ist, daß zwischen der Austrittsöffnung der unter Druck stehenden Gasquelle (30), die eine Gasversorgungseinrichtung (31) mit einer Austrittsöffnung und ein Gasregulierungsmittel (32), welches mit der Gasversorgungseinrichtung (31) unter selektiver Verbindung mit der Austrittsöffnung der Gasversorgungseinrichtung (31) in operativer Verbindung steht, enthält, und der Eintrittsöffnung des Vorratsbehälters für vorwärts treibbares Material (40) oder der Austrittsöffnung der Quelle für vorwärts treibbares Material (20) eine selektive Verbindung zur Verfügung gestellt wird, wobei die Quelle an vorwärts treibbarem Material ein Zuführmittel für vorwärts treibbares Material (21) aufweist, um eine selektive Verbindung zwischen der Austrittsöffnung des Zuführmittels für vorwärts treibbares Material (21) und einem Regulierungsmittel für vorwärts treibbares Material (22), das mit der Austrittsöffnung der Versorgungseinrichtung für vorwärts treibbares Material (21) und der Eintrittsöffnung des Vorratsbehälters für vorwärts treibbares Material (40) in operativer Verbindung steht, herzustellen.

2. Vorrichtung nach Anspruch 1, worin die Vorrichtung weiter umfaßt:
(e) ein Rückgewinnungsmittel (60) mit einer Eintrittsöffnung und wobei
das Mehrzweckventil (70) so ausgestaltet ist, daß zwischen entweder der Austrittsöffnung der unter Druck stehenden Gasquelle (30) oder der Austrittsöffnung der Quelle an vorwärts treibbarem Material (20) und der Eintrittsöffnung des Vorratsbehälters für vorwärts treibbares Material (40) eine selektive Verbindung hergestellt wird und zwischen entweder der Eintrittsöffnung des Zuführmittels (50) oder der Eintrittsöffnung des Rückgewinnungsmittels (60) und der Austrittsöffnung des Vorratsbehälters für vorwärts treibbares Material (40) eine selektive Verbindung hergestellt wird.

3. Vorrichtung nach Anspruch 2, worin die Vorrichtung weiter umfaßt:
(f) ein Zuführmittel (5) mit einer Eintrittsöffnung und einer Austrittsöffnung, worin die Eintrittsöffnung des Zuführmittels (50) mit der Austrittsöffnung des Vorratsbehälters für vorwärts treibbares Material (40) in Fluid-Verbindung steht, und
wobei das Mehrzweckventil (70) so ausgelegt ist, daß zwischen entweder der Austrittsöffnung der unter Druck stehenden Gasquelle (30) oder der Austrittsöffnung der Quelle an vorwärts treibbarem Material (20) und der Eintrittsöffnung des Vorratsbehälters für vorwärts treibbares Material (40) eine selektive Verbindung hergestellt wird und zwischen entweder der Eintrittsöffnung des Zuführmittels (50) oder der Eintrittsöffnung des Rückgewinnungsmittels (60) und der Austrittsöffnung des Vorratsbehälters für vorwärts treibbares Material (40) eine selektive Verbindung hergestellt wird.

4. Vorrichtung nach Anspruch 1, worin das Gasregulierungsmittel (32) ein Gasventil (32a) und einen Gasvorratsbehälter (32b) mit einer Eintrittsöffnung umfaßt, wobei das Gasventil (32a) zwischen der Austrittsöffnung der Gasversorgungseinrichtung (31) und der Eintrittsöffnung des Gasvorratbehälters (32b) eine selektive pneumatische Verbindung liefert.

5. Vorrichtung nach Anspruch 1, welche weiter umfaßt:
ein Temperaturregulierungsmittel (42), wobei das Temperaturregulierungsmittel (42) mit dem Vorratsbehälter für vorwärts treibbares Material (40) in operativer Verbindung steht.

6. Vorrichtung nach Anspruch 1, worin die Quelle an vorwärts treibbarem Materials (20) weiter umfaßt:
ein Rührmittel (24), wobei das Rührmittel (24) mit der Versorgungseinrichtung für vorwärts treibbares Material (21) in operativer Verbindung steht.

7. Vorrichtung nach Anspruch 3, wobei das Zuführmittel (50) eine Makro-Zielvorrichtung (51) umfaßt.

8. Vorrichtung nach Anspruch 3, wobei das Zuführmittel (50) eine Mikro-Zielvorrichtung (52) umfaßt.

9. Vorrichtung nach Anspruch 3, worin die Vorrichtung eine Geschwindigkeitsmeßvorrichtung (90) umfaßt.

10. Vorrichtung nach Anspruch 9, worin die Vorrichtung einen ersten Sensor (92) und einen zweiten Sensor (94) umfaßt.

11. Vorrichtung nach Anspruch 1, worin der Vorratsbehälter für vorwärts treibbares Material (40) mit einer von außen kontrollierten Kammer, die einen Vortriebsbereich festlegt, in Fluid-Verbindung steht.

12. Verfahren zum Einführen von biologischem Material in lebende Zellen, welches die folgenden Schritte umfaßt:
(a) ein bestimmtes Volumen eines Gases mit einem bestimmten Gasdruck zu liefern;
(b) eine bestimmte Menge eines vorwärts treibbaren Materials zu liefern, wobei das vorwärts treibbare Material in einem Trägermedium suspendiertes biologisches Material ist; und
(c) das vorwärts treibbare Material mit dem bestimmten Gasvolumen Inkontakt zu bringen, wobei das vorwärts treibbare Material auf ein bestimmtes Ziel hin beschleunigt wird.

13. Verfahren zum Einführen von biologischem Material in Zielmaterial, wobei das Verfahren die folgenden Schritte umfaßt:
(a) ein bestimmtes Volumen eines Gases in einem Gasbehälter bei einem vorbestimmten Druck zu liefern;
(b) einen Vorratsbehälter für vorwärts treibbares Material, eine bestimmte Menge eines vorwärts treibbaren Materials, welche eine Suspension eines biologischen Materials in einem Trägermedium umfaßt, zu liefern;
(c) das vorwärts treibbare Material mit dem bestimmten Volumen eines Gases Inkontakt zu bringen; und
(d) das vorwärts treibbare Material durch ein Zuführmittel auf das Zielmaterial hin zu beschleunigen.

14. Verfahren nach Anspruch 12, wobei das vorwärts treibbare Material auf ein bestimmtes Zielmaterial hin durch ein Zuführmittel (50) beschleunigt wird und das Zuführmittel (50) in einer Geschwindigkeit zwischen 322 und 1931 km/Std. (200 und 1200 Meilen/Std., 89,4 und 536,4 ms⁻¹) verlaßt.

15. Vorrichtung, welche das Folgende umfaßt:
(a) einen Vorratsbehälter für vorwärts treibbares Material (40) mit einer Eintrittsöffnung und einer Austrittsöffnung;
(b) ein Zuführmittel (50) mit einer Eintrittsöffnung und einer Austrittsöffnung, wobei die Eintrittsöffnung des Zuführmittels (50) mit der Austrittsöffnung des Vorratsbehälters (40) für vorwärts treibbares Material in Verbindung steht; und
(c) ein Mehrzweckventil (70), welches ein erstes Unterventil (70a) mit einer Austrittsöffnung und ein zweites Unterventil (70b) mit einer Eintrittsöffnung und einer Autrittsöffnung umfaßt, wobei die Austrittsöffnung des ersten Unterventils (70a) eine selektive Verbindung mit der Eintrittsöffnung des Vorratsbehälters für vorwärts treibbares Material (40) liefert und das zweite Unterventil (70b) eines elektive Verbindung zwischen der Austrittsöffnung des Vorratsbehälters für vorwärts treibbares Material (40) und der Eintrittsöffnung des Zuführmittels (50) liefert.

16. Vorrichtung nach Anspruch 15, welche weiter umfaßt:
(d) eine unter Druck stehende Gasquelle (30) mit einer Austrittsöffnung, wobei
das erste Unterventil (70a) des Mehrzweckventils (70) dazu befähigt ist eine selektive pneumatische Verbindung zwischen der Austrittsöffnung der unter Druck stehenden Gasquelle (30) und der Eintrittsöffnung des Vorratsbehälters für vorwärts treibbares Material (40) herzustellen, wobei die unter Druck stehende Gasquelle (30) bei Verbindung mit dem Vorratsbehälter für vorwärts treibbares Material (40) das Gas in den Vorratsbehälter für vorwärts treibbares Material (40) entleert und dies bei ausreichendem Druck, um das vorwärts treibbare Material zum Verlassen des Zuführmittels (50) zu bringen, wenn der Vorratsbehälter für vorwärts treibbares Material (40) mit dem Zuführmittel in Verbindung (50) steht, in einer Geschwindigkeit, welche ausreicht ein nicht-zelluläres biologisches Material in eine biologische Zelle einzuführen.

17. Vorrichtung nach Anspruch 16, worin die unter Druck stehende Gasquelle (30) bei Verbindung mit dem Vorratsbehälter für vorwärts treibbares Material (40) Gas unter einem ausreichendem Druck entleert, daß das vorwärts treibbare Material das zuführmittel (50) verläßt wenn der Vorratsbehälter für vorwärts treibbares Material (40) mit der Zuführvorrichtung in Verbindung steht, bei einer Geschwindigkeit zwischen 322 und 1931 km/Std. (200 und 1200 Meilen/Std., 89,4 und 536,4 ms⁻¹).

18. Vorrichtung nach Anspruch 15, welche weiter umfaßt:
(e) eine Quelle an vorwärts treibbarem Material (20) mit einer Austrittsöffnung, das erste Unterventil (70a) des Mehrzweckventils (70), welche eine selektive Verbindung zwischen der Austrittsöffnung der Quelle an vorwärts treibbarem Material (20) und der Eintrittsöffnung des Vorratsbehälters für vorwärts treibbares Material herstellt.

19. Verfahren zum Einführen von biologischem Material in Zielmaterial, wobei das Verfahren die folgenden Schritte umfaßt:
(a) eine Vorrichtung zur Verfügung zu stellen, welche aufweist:
(i) eine unter Druck stehende Gasquelle (30) mit einer Gas-Austrittsöffnung und eine Quelle an vorwärts treibbarem Material mit einer Aulaßöffnung;
(ii) einen Vorratsbehälter für vorwärts treibbares Material (40) mit einer Eintrittsöffnung und einer Austrittsöffnung;
(iii) ein Zuführmittel (50) mit einer Eintrittsöffnung und einer Austrittsöffnung, wobei die Eintrittsöffnung des Zuführmittel mit der Austrittsöffnung des Vorratsbehälters für vorwärts treibbares Material in Verbindung steht; und
(iv) ein Mehrzweckventil (70), worin das Mehrzweckventil (70) so ausgestaltet ist, daß es zwischen der Austrittsöffnung der unter Druck stehenden Gasquelle (30) und der Eintrittsöffnung des Vorratsbehälters für vorwärts treibbares Material eine selektive Verbindung liefert; und
(b) das Zielmaterial in einer bestimmten wirksamen Entfernung von der Austrittsöffnung der Zuführvorrichtung anzuordnen; und
(c) die Zuführvorrichtung auf das Zielmaterial auszurichten;
(d) das Mehrzweckventil so einzustellen, daß eine Fluid-Verbindung zwischen der Versorgungseinrichtung und dem Vorratsbehälter für vorwärts treibbares Material hergestellt wird, wobei ein gewähltes Volumen an vorwärts treibbarem Material in den Vorratsbehälter für vorwärts treibbares Material gelangt; und
(e) das Mehrzweckventil so einzustellen, daß eine pneumatische Verbindung zwischen der Austrittsöffnung der Gasversorgungseinrichtung und der Eintrittsöffnung des Vorratsbehälters für vorwärts treibbares Material und der Eintrittsöffnung des Zuführmittels geliefert wird, wobei ein gewähltes Gasvolumen auf das vorwärts treibbare Material in dem Vorratsbehälter für vorwärts treibbares Material trifft.

20. Verfahren nach Anspruch 19, wobei die Geschwindigkeit des vorwärts treibbaren Materials durch eine Geschwindigkeitsmeßvorrichtung verfolgt wird.

21. Verfahren nach Anspruch 20, welches den Schritt (f) umfaßt, bei dem das Ventil selektiv so eingestellt wird, daß der Durchgang des Gases aus dem Gasbehälter durch das Ventil in die Versorgungseinrichtung verhindert wird und daß eine Fluid-Verbindung zwischen einer Spritze und dem Überfluß-Vorratsbehälter für vorwärts treibbares Material hergestellt wird.

## Revendications

1. Appareil comprenant :
(a) une source (30) de gaz sous pression qui comprend une sortie de gaz et une source de matière propulsable comportant une sortie,
(b) un réservoir (40) de matière propulsable comportant une entrée et une sortie, caractérisé en ce qu'il comprend en outre :
(c) une vanne polyvalente (70), sachant que la vanne polyvalente (70) est d'une conception qui offre une communication sélective entre la sortie de la source (30) de gaz sous pression, qui comprend un moyen (31) d'alimentation en gaz comportant une sortie et un moyen (32) de régulation du gaz fonctionnant en association avec le moyen (31) d'alimentation en gaz pour donner une communication sélective avec la sortie du moyen (31) d'alimentation en gaz et l'entrée du réservoir (40) de matière propulsable, ou la sortie de la source (20) de matière propulsable qui comporte un moyen (21) d'alimentation en matière propulsable pour donner une communication sélective entre la sortie du moyen (21) d'alimentation en matière propulsable et un moyen (22) de régulation de la matière propulsable qui agit en association avec la sortie du moyen (21) d'alimentation en matière propulsable, et l'entrée du réservoir (40) de matière propulsable.

2. Appareil selon la revendication 1, sachant que ledit appareil comprend en outre :
(e) un moyen de récupération (60) avec une entrée, et que la vanne polyvalente (70) est d'une conception qui offre une communication sélective entre soit la sortie de la source (30) de gaz sous pression soit la sortie de la source de matière propulsable et l'entrée du réservoir (40) de matière propulsable et qui offre une communication sélective entre soit l'entrée du moyen de décharge (50) soit l'entrée du moyen de récupération (60) et la sortie du réservoir (40) de matière propulsable.

3. Appareil selon la revendication 2, sachant que ledit appareil comprend en outre :
(f) un moyen de décharge (50) comportant une entrée et une sortie, sachant que l'entrée du moyen de décharge (50) est en communication assurant le passage des fluides avec la sortie du réservoir (40) de matière propulsable, et
que la vanne polyvalente (70) est d'une conception qui offre une communication sélective entre soit la sortie de la source (30) de gaz sous pression soit la sortie de la source (20) de matière propulsable et l'entrée du réservoir (40) de matière propulsable, et qui offre une communication sélective entre soit l'entrée du moyen de décharge (50) soit l'entrée du moyen de récupération (60) et la sortie du réservoir (40) de matière propulsable.

4. Appareil selon la revendication 1, dans lequel le moyen (32) de régulation du gaz comprend un robinet de gaz (32a) et un réservoir de gaz (32b) avec une entrée, sachant que le robinet de gaz (32a) offre une communication pneumatique sélective entre la sortie du moyen (31) d'alimentation en gaz et l'entrée du réservoir de gaz (32b).

5. Appareil selon la revendication 1, comprenant en outre un moyen (42) de régulation de la température, ledit moyen (42) de régulation de la température étant en association opérationnelle avec le réservoir (40) de matière propulsable.

6. Appareil selon la revendication 1, dans lequel la source (20) de matière propulsable comprend en outre un moyen d'agitation (24), ledit moyen d'agitation (24) étant en association opérationnelle avec le moyen (21) d'alimentation en matière propulsable.

7. Appareil selon la revendication 3, dans lequel le moyen de décharge (50) comprend un moyen de macropointage (51).

8. Appareil selon la revendication 3, dans lequel le moyen de décharge (50) comprend un moyen de micropointage (52).

9. Appareil selon la revendication 3, sachant que ledit appareil comprend un moyen (90) de détection de la vitesse.

10. Appareil selon la revendication 9, sachant que ledit appareil comprend un premier capteur (92) et un second capteur (94).

11. Appareil selon la revendication 1, dans lequel le réservoir (40) de matière propulsable est en communication assurant le passage des fluides avec une chambre à atmosphère contrôlée, ladite chambre définissant une région de propulsion (80).

12. Procédé pour introduire de la matière biologique dans des cellules vivantes, ledit procédé comprenant les étapes consistant à :
(a) préparer un volume prédéterminé d'un gaz ayant une pression de gaz sélectionnée,
(b) préparer une quantité prédéterminée de matière propulsable, sachant que la matière propulsable est un matériau biologique en suspension dans un milieu porteur, et
(c) mettre en contact ladite matière propulsable avec le volume prédéterminé de gaz, sachant que la matière propulsable est accélérée au niveau d'une cible sélectionnée.

13. Procédé pour introduire de la matière biologique dans une matière cible, ledit procédé comprenant les étapes consistant à :
(a) préparer un volume prédéterminé d'un gaz dans un réservoir de gaz à des pressions prédéterminées,
(b) préparer, dans un réservoir de matière propulsable, une quantité prédéterminée de matière propulsable qui contient une suspension d'un matériau biologique dans un milieu porteur,
(c) mettre en contact ladite matière propulsable avec le volume prédéterminé de gaz, et
(d) accélérer ladite matière propulsable au niveau de la matière cible à l'aide d'un moyen de décharge.

14. Procédé selon la revendication 12, dans lequel la matière propulsable est accélérée au niveau de la cible sélectionnée à l'aide d'un moyen de décharge (50) et sort du moyen de décharge (50) à une vitesse comprise entre 322 et 1931 km/heure (entre 200 et 1200 miles par heure), soit entre 89,4 et 536,7 ms⁻¹.

15. Appareil comprenant :
(a) un réservoir (40) de matière propulsable comportant une entrée et une sortie,
(b) moyen de décharge (50) avec une entrée et une sortie, l'entrée du moyen de décharge (50) étant en communication avec la sortie du réservoir (40) de matière propulsable, et
(c) une vanne polyvalente (70) qui comprend une première unité formant vanne (70a) avec une sortie et une seconde unité formant vanne (70b) avec une entrée et une sortie, la sortie de la première unité formant vanne (70a) permettant une communication sélective avec l'entrée du réservoir (40) de matière propulsable et la seconde unité formant vanne (70b) permettant une communication sélective entre la sortie du réservoir (40) de matière propulsable et l'entrée du moyen de décharge (50).

16. Appareil selon la revendication 15, comprenant en outre :
(d) une source (30) de gaz sous pression avec une sortie de gaz,
la première unité formant vanne (70a) de la vanne polyvalente (70) étant capable d'offrir une communication pneumatique sélective entre la sortie de la source (30) de gaz sous pression et l'entrée du réservoir (40) de matière propulsable,
quand elle est en communication avec le réservoir (40) de matière propulsable, la source (30) de gaz sous pression décharge du gaz dans le réservoir (40) de matière propulsable sous une pression suffisante pour amener une matière propulsable à sortir du moyen de décharge (50), quand le réservoir (40) de matière propulsable est en communication avec le moyen de décharge, à une vitesse efficace pour faire pénétrer dans une cellule vivante un matériau biologique étranger à cette cellule.

17. Appareil selon la revendication 16, dans lequel la source (30) de gaz sous pression, quand elle est en communication avec le réservoir (40) de matière propulsable, décharge du gaz sous une pression suffisante pour amener une matière propulsable à sortir du moyen de décharge (50), quand le réservoir (40) de matière propulsable est en communication avec le moyen de décharge, à une vitesse comprise entre 322 et 1931 km/heure (entre 200 et 1200 miles par heure), soit entre 89,4 et 536,7 ms⁻¹.

18. Appareil selon la revendication 15, comprenant en outre :
(e) une source (20) de matière propulsable avec une sortie,
la première unité formant vanne (70a) de la vanne polyvalente (70) offrant une communication sélective entre la sortie de la source (20) de matière propulsable et l'entrée du réservoir (40) de matière propulsable.

19. Procédé pour introduire de la matière biologique dans une matière cible, ledit procédé comprenant les étapes consistant à :
(a) préparer un appareil qui comprend :
(i) une source (30) de gaz sous pression qui comprend une sortie de gaz et une source de matière propulsable comportant une sortie,
(ii) un réservoir (40) de matière propulsable comportant une entrée et une sortie,
(iii) un moyen de décharge (50) comportant une entrée et une sortie, sachant que l'entrée du moyen de décharge est en communication avec la sortie du réservoir de matière propulsable, et
(iv) une vanne polyvalente (70), sachant que la vanne polyvalente (70) est d'une conception qui offre une communication sélective entre la sortie de la source (30) de gaz sous pression et l'entrée du réservoir (40) de matière propulsable,
(b) mettre en place la matière cible à une distance utile sélectionnée de la sortie du moyen de décharge,
(c) pointer le moyen de décharge sur la matière cible,
(d) régler la vanne polyvalente pour qu'elle offre une communication assurant le passage des fluides entre le moyen d'alimentation en matière propulsable et le réservoir de matière propulsable, ce qui fait qu'un volume sélectionné de matière propulsable entre dans le réservoir de matière propulsable, et
(e) régler la vanne polyvalente pour qu'elle offre une communication pneumatique entre la sortie du moyen d'alimentation en gaz et l'entrée du réservoir de matière propulsable, ainsi qu'entre la sortie du réservoir de matière propulsable et l'entrée du moyen de décharge, ce qui fait qu'un volume sélectionné de gaz vient en contact avec la matière propulsable dans le réservoir de matière propulsable.

20. Procédé selon la revendication 19, dans lequel la vitesse de la matière propulsable après sa sortie du réservoir de matière propulsable est surveillée par un moyen de mesure de la vitesse.

21. Procédé selon la revendication 20, comprenant une étape (f) qui consiste à régler la vanne polyvalente de manière sélective pour bloquer le passage du gaz du réservoir de gaz au moyen de décharge par la vanne et pour offrir une communication assurant le passage des fluides entre une seringue et un réservoir de trop-plein de matière propulsable.
